(19) **Europäisches Patentamt / European Patent Office / Office européen des brevets**

(11) **EP 2 917 333 B1**

(12) # EUROPEAN PATENT SPECIFICATION

(45) Date of publication and mention of the grant of the patent:
**10.01.2018 Bulletin 2018/02**

(21) Application number: **13795380.8**

(22) Date of filing: **08.11.2013**

(51) Int Cl.:
**C12N 1/12** *(2006.01)*    **A01G 33/00** *(2006.01)*

(86) International application number:
**PCT/US2013/069046**

(87) International publication number:
**WO 2014/074769 (15.05.2014 Gazette 2014/20)**

(54) **METHODS OF CULTURING MICROORGANISMS IN NON-AXENIC MIXOTROPHIC CONDITIONS AND CONTROLLING BACTERIAL CONTAMINATION IN THE CULTURES USING ACETATE AND / OR OXIDIZING AGENTS**

VERFAHREN ZUR ZÜCHTUNG VON MIKROORGANISMEN UNTER NICHT-AXENISCHEN MIXOTROPHEN BEDINGUNGEN UND ZUR STEUERUNG DER BAKTERIELLEN VERUNREINIGUNG IN DEN KULTUREN MIT ACETAT UND/ODER OXIDATIONSMITTELN

PROCÉDÉS DE CULTURE DE MICROORGANISMES DANS DES CONDITIONS MIXOTROPHES NON AXÉNIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.11.2012 US 201261724710 P**
**15.03.2013 US 201361798969 P**
**15.03.2013 US 201361799151 P**
**13.09.2013 US 201361877894 P**

(43) Date of publication of application:
**16.09.2015 Bulletin 2015/38**

(73) Proprietor: **Heliae Development LLC**
**Gilbert, AZ 85297 (US)**

(72) Inventors:
- **GANUZA, Eneko**
  **Phoenix, AZ 85014 (US)**
- **LICAMELE, Jason, David**
  **Scottsdale, AZ 85225 (US)**
- **TONKOVICH, Anna, Lee**
  **Gilbert, AZ 85297 (US)**

(74) Representative: **Bassil, Nicholas Charles et al**
**Kilburn & Strode LLP**
**Lacon London**
**84 Theobalds Road**
**London WC1X 8NL (GB)**

(56) References cited:
WO-A1-01/04338          WO-A1-2013/096682
WO-A2-2009/073822       WO-A2-2012/109375
US-A- 3 444 647         US-A1- 2009 304 810
US-A1- 2010 330 653     US-A1- 2011 190 522
US-A1- 2012 011 620     US-A1- 2013 164 796

- **MAKOTO KITANO ET AL: "Changes in eicosapentaenoic acid content of Navicula saprophila, Rhodomonas salina and Nitzschia sp. under mixotrophic conditions", JOURNAL OF APPLIED PHYCOLOGY, KLUWER, DORDRECHT, NL, vol. 9, no. 6, 1 December 1997 (1997-12-01), pages 559-563, XP008162043, ISSN: 0921-8971, DOI: 10.1023/A:1007908618017**
- **SAITHONG P ET AL: "Prevention of bacterial contamination using acetate-tolerant Schizosaccharomyces pombe during bioethanol production from molasses", JOURNAL OF BIOSCIENCE AND BIOENGINEERING, ELSEVIER, AMSTERDAM, NL, vol. 108, no. 3, 1 September 2009 (2009-09-01), pages 216-219, XP026436941, ISSN: 1389-1723 [retrieved on 2009-08-05]**
- **NASIR KURESHY ET AL: "Effect of Ozone Treatment on Cultures of Nannochloropsis oculata, Isochrysis galbana, and Chaetoceros gracilis", JOURNAL OF THE WORLD AQUACULTURE SOCIETY, vol. 30, no. 4, 1 December 1999 (1999-12-01), pages 473-480, XP55119424,**

EP 2 917 333 B1

**(Cont. next page)**

- CHENG Y L ET AL: "Dispersed ozone flotation of Chlorella vulgaris", BIORESOURCE TECHNOLOGY, ELSEVIER BV, GB, vol. 101, no. 23, 1 December 2010 (2010-12-01), pages 9092-9096, XP027213126, ISSN: 0960-8524 [retrieved on 2010-08-02]
- KOBAYASHI M ET AL: "Growth and astaxanthin formation of Haematococcus pluvialis in heterotrophic and mixotrophic conditions", JOURNAL OF FERMENTATION AND BIOENGINEERING, SOCIETY OF FERMENTATION TECHNOLOGY, JP, vol. 74, no. 1, 1 January 1992 (1992-01-01), pages 17-20, XP023595533, ISSN: 0922-338X, DOI: 10.1016/0922-338X(92)90261-R [retrieved on 1992-01-01]
- GARCIA-OCHOA F ET AL: "Bioreactor scale-up and oxygen transfer rate in microbial processes: An overview", BIOTECHNOLOGY ADVANCES, ELSEVIER PUBLISHING, BARKING, GB, vol. 27, no. 2, 1 March 2009 (2009-03-01), pages 153-176, XP025873361, ISSN: 0734-9750, DOI: 10.1016/J.BIOTECHADV.2008.10.006 [retrieved on 2008-11-12]

**Description**

**CROSS REFERENCE TO RELATED APPLICATIONS**

This application claims the benefit of U.S. Provisional Application No. 61/724,710, filed November 9, 2012, entitled Methods of Culturing Microorganisms in Mixotrophic Conditions; U.S. Provisional Application No. 61/798,969, filed March 15, 2013, entitled Mixotrophy Systems and Methods; U.S. Provisional Application No. 61/799,151, filed March 15, 2013, entitled Mixotrophy Technology; and U.S. Provisional Application No. 61/877,894, filed September 13, 2013, entitled Methods of Culturing Microorganisms in Non-Axenic Mixotrophic Conditions.

**BACKGROUND**

Microorganisms, such as but not limited to microalgae and cyanobacteria, have gained attention as a viable source for food, fuel, fertilizers, cosmetics, chemicals, and pharmaceuticals due to the ability to grow rapidly and in a variety of conditions, such as a wastewater medium. Each microalgal and cyanobacteria species have a different protein, mineral, and fatty acid profile which makes some species better sources for certain products than other species. Different microalgal and cyanobacteria species may use different sources of energy and carbon. Phototrophic microorganisms use light energy, as well as inorganic carbon (e.g., carbon dioxide), to carry out metabolic activity. Heterotrophic microorganisms do not use light as an energy source and instead use an organic carbon source for energy and carbon to carry out metabolic activity. Mixotrophic microorganisms can use a mix of energy and carbon sources, including light, inorganic carbon (e.g., carbon dioxide) and organic carbon. The versatility of mixotrophic microorganisms, which are capable of using a variety of energy and carbon sources, provides the potential to thrive in conditions challenging for obligate phototrophs or heterotrophs, and reduce the effects of biomass loss through respiration of cell materials. Additionally, the growth of the mixotrophic microorganisms attributable to the ratio of phototrophic and heterotrophic metabolisms results in different nutrients limiting the growth of the microorganisms than in pure a phototrophic or heterotrophic culture.

Even with the versatility of mixotrophic microorganisms to use different energy and carbon sources, mixotrophic cultures face their own set of challenges. Heterotrophic cultures using an organic carbon source are maintained under axenic or sterile conditions to prevent contamination with bacteria, fungi, or other unwanted contaminating species that use the organic carbon source as a feed source. These heterotrophic microorganism cultures usually comprise closed, sealed and autoclavable bioreactor systems, resulting in a higher cost and complexity than simple open bioreactor systems. Phototrophic microorganism cultures may be grown outside in non-axenic conditions for exposure to natural light at a cost lower that the closed heterotrophic bioreactor system, and do not use an organic carbon source (which may provide for a feed source for contaminating bacteria).

A mixotrophic microorganism culture which utilizes light may be grown outdoors in an open bioreactor system for access to natural light, but also includes an organic carbon source which creates the increased potential for contamination by bacteria and other contaminating organisms in the culture due to the ability of bacteria and fungi to use the organic carbon source to grow at a faster rate than the mixotrophic microalgae or cyanobacteria. Alternatively, a mixotrophic microorganism culture may be grown indoors in the presence of ambient light or augmented light from artificial light sources, such as light emitting diodes (LED) and fluorescent lights, but may still experience a similar potential for contamination due to the presence of an organic carbon source.

Additionally, contaminating bacteria may affect microorganism product formation (e.g., lipids, pigments, proteins) and growth. The fraction of microorganism growth attributed to the heterotrophic metabolism may also be dictated by the microorganisms present in the culture. The proliferation of contaminating bacteria and other contamination organisms in a mixotrophic microorganism culture has proven to be detrimental to the production of microalgae and cyanobacteria if the contaminating bacteria population is not controlled and is allowed to consume resources needed by the microalgae and cyanobacteria. Therefore, there is a need in the art for a method of efficiently culturing microalgae and cyanobacteria under non-axenic mixotrophic conditions which control the contamination and maintain the culture nutrients at levels to maximize growth of the microalgae and cyanobacteria.

The current disclosure relates to a method for mixotrophic growth of microorganisms in non-axenic conditions that use an acetic acid/pH auxostat to supply an organic carbon source and control the pH level of the culture. Further described are alternative methods with different sources of carbon and also operate in non-axenic conditions. The details not taught in the prior art that allow this method to be run in non-axenic conditions, such as open ponds, while maintaining control over bacterial contaminants are also described herein.

In the prior art, the pH auxostat cultivation system was first reported by two different research groups in the 1960's (Bungay, 1972; Watson 1969) and a detailed development was presented by Martin and Hempfling (1976). While this research concerned bacteria and yeast cultures, Ratledge and co-workers (2001) reported a microalgae based acetic acid/pH auxostat in heterotrophic conditions. *Crypthecodinium cohnii* cultured heterotrophically by Ratledge using acetic acid as the organic carbon source showed an improvement in heterotrophic growth and lipid accumulation with an acetic acid/pH auxostat cultivation method. Although Ratledge used the acetic acid/pH-auxostat to grow microalgae, the system was limited to heterotrophic species in a closed, fermentation system, which does not face the same challenges regarding contamination control as an open, mixotrophic system. Mixotrophic monoalgal cultures in open ponds have been reported with organic carbon sources other than acetic acid, but the bacterial population of the culture was not controlled or addressed. WO 2012/109375 A2 describes the utilization of glucose as the organic carbon source in a *Chlorella* culture grown mixotrophically in an open pond at a low density before harvesting the biomass for lipid production in a heterotrophic system. Due to the short time in the mixotrophic stage, the culture does not experience the same contamination challenges as a purely mixotrophic culture producing high density biomass and lipids over a longer time period.

Also, the role of acetic acid as a bactericide is known in the art (Huang et al, 2011; Roe et al, 2002), but so far it has not been applied to control bacterial populations in large scale mixotrophic microalgae cultures. The utilization of acetic acid in mixotrophic microalgae cultures has generally been in lab scale experiments, where standard laboratory conditions assume axenic operation and the bacterial population of the culture was not addressed (Yeh et al. 2011). In US 3,444,647 Takashi discloses mixotrophic cultures of *Chlorella* in flask cultures containing different carbon sources and observed lower bacterial levels associated with the culture comprising acetic acid, but $CO_2$ was used to control the pH level within the flask cultures and not the acetic acid. Most importantly US 3,444,647 does not disclose methods to control contaminating bacteria in small or large scale microalgae cultures, or methods to maximize biomass production of the microalgae in non-axenic mixotrophic growth conditions. The inventive method hereby described innovates on the prior art by creating a stable process that controls heterotrophic contamination while boosting microalgae productivities to higher levels (e.g., 3 g/L d) than were previously described for an illuminated culture.

## SUMMARY

Aspects of the invention for which protection is sought are as defined in the claims. The invention provides a method of culturing *Chlorella* in non-axenic mixotrophic culture conditions, comprising:

(a) inoculating an aqueous culture medium with a culture comprising *Chlorella* in an open culturing vessel;

(b) supplying light comprising photosynthetically active radiation (PAR) in a photoperiod in the range of 10-16 hours of light for each 24 hour day;

(c) activating a pH auxostat system with an initial concentration of sodium acetate in the range of 0.05-10 g/L in the aqueous culture medium to start supplying acetic acid, at a concentration in the range of 15-50% (v/v) to the culture comprising *Chlorella,* wherein the pH auxostat system operates with in the range of 6-9, and the temperature ranges from 10 °C to 30 °C; and

(d) maintaining a dissolved oxygen (DO) concentration in the range of 1-6 mg $O_2$/Liter.

The method may further comprise controlling temperature of the culture with heating and cooling to maintain the temperature within the range of 10-30°C.

The supplied acetic acid may be mixed with a second organic carbon component comprising propionic acid. The supplied acetic acid and propionic acid may be mixed in an acetic acid:propionic acid ratio in the range of 10:0.01 to 10:2.

The supplied acetic acid may be mixed with at least one additional nutrient comprising at least one selected from the group consisting of nitrates and phosphates. The at least one additional nutrient may comprise $NO_3$ wherein the acetic acid and $NO_3$ are mixed in an acetic acid: $NO_3$ ratio of 10:0.5 to 10:2.

The method may further comprise harvesting *Chlorella* from the culture on a periodic basis once the density of *Chlorella* in the culture reaches a threshold density of 5 g/L.

The initial concentration of sodium acetate may be in the range of 0.1-6 g/L.

The initial concentration of sodium acetate may be supplied for the first 1-5 days after the culture is inoculated. The disclosure relates generally to systems and methods for culturing microorganisms mixotrophically in non-axenic conditions. In particular, described herein is how to optimize growth and control contamination in a culture with an organic carbon source, oxidative agents, and gas transfer.

A method of culturing microorganisms in non-axenic mixotrophic conditions may comprise: inoculating an aqueous culture medium with a culture of microorganisms comprising at least some contaminating bacteria in a culturing vessel; supplying the culture of microorganisms with at least some light; supplying the culture of microorganisms with an organic carbon source comprising an organic acid; and wherein the culture of microorganisms maintains a level of contaminating bacteria below 25% of a total cell count of the culture and a microorganism yield of at least 50 g/m$^2$ day.

As also described herein, the microorganism can comprise at least one microorganism of the genus selected from the group consisting of: *Chlorella, Anacystis, Synechococcus, Synechocystis, Neospongiococcum, Chlorococcum, Phaeodactylum, Spirulina, Micractinium, Haematococcus, Nannochloropsis,* and *Brachiomonas.* The contaminating bacteria may comprise at least one selected from the group consisting of: *Achromobacter* sp., *Acidovorax* sp., *Aeromonas* sp., *Agrobacterium* sp., *Alteromonas* sp., *Aquaspirillum* sp., *Azospirillum* sp., *Azotobacter* sp., *Bergeyella* sp., *Brochothrix* sp., *Brumimicrobium* sp., *Burkholderia* sp., *Caulobacter* sp., *Cellulomonas* sp., *Chryseobacterium* sp., *Curtobacterium* sp., *Delftia* sp., *Empedobacter* sp., *Enterobacter* sp., *Escherichia* sp., *Flavobacterium* sp., *Marinobacter* sp., *Microbacterium* sp., *Myroides* sp., *Paracoccus* sp., *Pedobacter* sp., *Phaeobacter* sp., *Pseudoalteromonas* sp., *Pseudomonas* sp., *Rahnella* sp., *Ralstonia* sp., *Rhizobium* sp., *Rhodococcus* sp., *Roseomonas* sp., *Staphylococcus* sp., *Stenotrophomonas* sp., *Vibrio* sp., and *Zobelliae* sp.

As described herein, the organic acid of the organic carbon source may comprise 0.5-50% acetic acid. The culture of microorganisms may maintain a level of contaminating bacteria below 20%, 10%, or 5% of a total cell count of the culture. As described herein, the organic acid is combined with at least one other nutrient and supplied to the culture medium in combination, the at least one other nutrient comprising at least one selected from the group consisting of: nitrates, phosphates, iron, cobalt, copper, sodium, molybdenum, manganese, zinc, salts, and silica. The method may further comprise supplying a least one oxidizing agent to the microorganism culture, the at least one oxidizing agent comprising at least one selected from the group consisting of: ozone, hydrogen peroxide, chlorine, chlorite, chlorate, hypochlorite, nitric acid, chromium, permanganate, silver oxide, and bromine.

As described herein, the organic carbon source is supplied to the culture by a pH auxostat system when a measured pH level of the culture reaches a threshold level. The pH threshold level may be 7.5. The pH auxostat system can maintain a substantially constant pH level in the microorganism culture. The pH auxostat may maintain a pH level within a defined hysteresis range that inhibits proliferation of contaminating bacteria in the microorganism culture. The organic carbon source may be supplied to the culture until a measured dissolved oxygen level of the culture reaches a critical level below 2 mg O$_2$/L.

As described herein, the aqueous culture medium comprises an initial concentration of sodium acetate, sodium hydroxide, or potassium hydroxide between 0.1 and 6 g/L. As described herein, the at least some light is supplied to the microorganism culture for a photoperiod of 10-16 , less than 15, or more than 15 hours per day. The at least some light may be natural light, artificial light, or a combination thereof. The at least some light may comprise at least one specific wavelength spectrum from the group consisting of: violet (about 380-450 nm), blue (about 450-495 nm), green (about 495-570 nm), yellow (570-590 nm), orange (about 590-620 nm), red (about 620-750 nm), and far red (about 700-800 nm). The culturing vessel may be an open vessel disposed outdoors.

A method of controlling bacterial contamination in a mixotrophic culture of microorganisms in non-axenic conditions may comprise: inoculating an aqueous culture medium with a culture of microorganisms comprising at least some contaminating bacteria in a culturing vessel; supplying the culture of microorganisms with at least some light; supplying the culture of microorganisms with an organic carbon source; supplying the culture of microorganisms with an oxidizing agent; and wherein the culture of microorganisms maintains a level of contaminating bacteria below 25% of total cell count of the microorganism culture. The oxidative agent may comprise at least one selected from the group consisting of: ozone, hydrogen peroxide, chlorine, chlorite, chlorate, hypochlorite, nitric acid, chromium, permanganate, silver oxide, and bromine.

As described herein, ozone may be supplied in concentrations between 0.1 and 2.0 mg/L. The oxidizing agent may be supplied to the culture of microorganisms through at least one of a sparger and a venturi injector. As described herein, the microorganism comprises at least one microorganism of a genus selected from the group consisting of: *Agmenellum, Amphora, Anabaena, Anacystis, Apistonema, Arthrospira (Spirulina), Botryococcus, Brachiomonas, Chlamydomonas, Chlorella, Chloroccum, Cruciplacolithus, Cylindrotheca, Coenochloris, Cyanophora, Cyclotella, Dunaliella, Emiliania, Euglena, Extubocellulus, Fragilaria, Galdieria, Goniotrichium, Haematococcus, Halochlorella, Isochyrsis, Leptocylindrus, Micractinium, Melosira, Monodus, Nostoc, Nannochloris, Nannochloropsis, Navicula, Neospongiococcum, Nitzschia., Odontella, Ochromonas, Ochrosphaera, Pavlova, Picochlorum, Phaeodactylum, Pleurochyrsis, Porphyridium, Poteriochromonas, Prymnesium, Rhodomonas, Scenedesmus, Skeletonema, Spumella, Stauroneis, Stichococcus, Auxenochlorella, Cheatoceros, Neochloris, Ocromonas, Porphiridium, Synechococcus, Synechocystis, Tetraselmis Thraustochytrids,* and *Thalassiosira.*

As described herein, the organic carbon source comprises at least one selected from the group consisting of: acetate, acetic acid, ammonium linoleate, arabinose, arginine, aspartic acid, butyric acid, cellulose, citric acid, ethanol, fructose, fatty acids, galactose, glucose, glycerol, glycine, lactic acid, lactose, maleic acid, maltose, mannose, methanol, molasses, peptone, plant based hydrolyzate, proline, propionic acid, ribose, sacchrose, partial or complete hydrolysates of starch, sucrose, tartaric, TCA-cycle organic acids, thin stillage, urea, industrial waste solutions, and yeast extract.

Also described herein is a method of culturing microorganisms in non-axenic mixotrophic conditions, which comprises: inoculating an aqueous culture medium with a culture of microorganisms comprising at least some contaminating bacteria in a culturing vessel; supplying the culture of microorganisms with at least some light; supplying the culture of microorganisms with an organic carbon source; supplying the culture with a gas comprising oxygen; and wherein the gas is supplied to the culture at a $k_La$ of at least 2.40X10-3 s-1, and results in a productivity rate for the microorganism of at least 0.4 g/L d. The $k_La$ may be between 2.70X10$^{-3}$ s$^{-1}$ and 21 s$^{-1}$. The productivity rate for the microorganisms may be between 0.5 g/L d and 50 g/L d.

As described herein, the gas is supplied to the culture of microorganisms by at least one selected from the group consisting of a gas injector, porous diffuser, micropore diffuser, gas permeable membrane, microbubble generator, venturi injection, and microbubble fluidic oscillator. The organic carbon source may comprise acetic acid and the aqueous culture medium comprises an initial concentration and supply for the first 1-5 days of sodium acetate, sodium hydroxide, or potassium hydroxide.

Also described herein is a method of growing microorganisms mixotrophically comprising providing a culture of microorganisms in an aqueous culture medium in a culturing vessel, the microorganisms being capable of utilizing light and organic carbon as energy sources; supplying at least some light to the culture of microorganisms; and supplying an organic carbon source comprising acetic acid and an oxaloacetate promoter to the culture of microorganisms. The organic acid may comprise at least one form the group consisting of acetic acid, acetate, and acetic anhydride. The oxaloacetate promoter may comprise at least one from the group consisting of propionic acid, valine, isoleucine, threonine, and methionine. The ratio of organic acid to oxaloacetate promoter may range from 10:0.01 to 10:2.

## BRIEF DESCRIPTION OF FIGURES

FIG. 1 is a comparative graph showing the productivity in cell dry weight and lipids as a percentage of cell dry weight between photoautotrophic and mixotrophic cultures.

FIG. 2 is a comparative graph showing the productivity in cell dry weight and lipids as a percentage of cell dry weight between photoautotrophic and mixotrophic cultures.

FIG. 3 is a comparative graph showing the NaNO3 uptake between photoautotrophic and mixotrophic cultures, and acetate uptake for a mixotrophic culture.

FIG. 4 is a comparative graph showing the $NaNO_3$ uptake between photoautotrophic and mixotrophic cultures, and acetate uptake for a mixotrophic culture.

FIG. 5 is a comparative graph showing the residual acetate for two mixotrophic cultures.

FIG. 6 is a comparative graph showing the productivity in cell dry weight for photoautotrophic and mixotrophic cultures with 24 and 14 hour photoperiods.

FIG. 7 is a comparative graph showing the nitrate uptake for photoautotrophic and mixotrophic cultures with 24 and 14 hour photoperiods.

FIG. 8 is a comparative graph showing the acetate uptake for mixotrophic cultures with 24 and 14 hour photoperiods.

FIG. 9 is a comparative graph showing the dissolved oxygen level for photoautotrophic and mixotrophic cultures with 24 and 14 hour photoperiods.

FIG. 10 is a comparative graph showing the productivity in ash free cell dry weight for photoautotrophic and mixotrophic cultures with 24 and 0 hour photoperiods, and dissolved oxygen concentration.

FIG. 11 is a comparative graph showing the acetic acid addition for mixotrophic and heterotrophic cultures.

FIG. 12 is a comparative graph showing the $NaNO_3$ uptake for photoautotrophic and mixotrophic cultures with 24 and 0 hour photoperiods.

FIG. 13 is a comparative graph showing the productivity in cell dry weight for two mixotrophic cultures.

FIG. 14 is a comparative graph showing dissolved oxygen level and maximum temperature for two mixotrophic cultures.

FIG. 15 is a comparative graph showing the productivity in cell dry weight, residual $NaNO_3$ and dissolved oxygen level for a mixotrophic culture.

FIG. 16 is a comparative graph showing the productivity in cell dry weight, residual $NaNO_3$ and dissolved oxygen level for a mixotrophic culture.

FIG. 17 is a comparative graph showing the bacteria counts and temperature for two mixotrophic cultures.

FIG. 18 is a comparative graph showing the productivity in ash free cell dry weight of a culture which transitioned from mixotrophic to photoautotrophic conditions.

FIG. 19 is a comparative graph showing the productivity in ash free cell dry weight for mixotrophic and photo autotrophic cultures of initial sodium acetate concentrations of 2 and 0 g/L.

FIG. 20 is a comparative graph showing the acetic acid addition for mixotrophic cultures of initial sodium acetate concentrations of 2 and 0 g/L.

FIG. 21 is a comparative graph showing the effects of $H_2O_2$ applications to mediums containing glucose and acetate.

FIG. 22 is a comparative graph showing the effects of $H_2O_2$ applications to mediums containing glucose and acetate.

FIG. 23 is a comparative graph showing the productivity in ash free dry weight for two mixotrophic cultures.

FIG. 24 is a comparative graph showing the yield in $g/m^2$ day for two mixotrophic cultures.

FIG. 25 is a comparative graph showing the volumetric growth rate for two mixotrophic cultures.

FIG. 26 is a comparative graph showing the productivity in $g/m^2$ day and acetic acid consumption for two mixotrophic cultures.

FIG. 27 is a comparative graph showing the nitrate consumption for two mixotrophic cultures.

FIG. 28 is a comparative graph showing the percentage of bacteria to *Chlorella* for two mixotrophic cultures.

FIG. 29 is a comparative graph showing the oxygen concentration for two mixotrophic cultures.

FIG. 30 is a comparative graph showing the oxygen concentration for a mixotrophic culture.

FIG. 31 is a comparative graph showing the bacterial concentration and live microalgae cells of a sonication treated microalgae culture.

FIG. 32 is a graph showing a mixotrophic microalgae culture concentration and volumetric growth rate (g/L) over time.

FIG. 33 is a graph showing a mixotrophic microalgae culture areal growth rate ($g/m^2$) over time.

FIG. 34 is a graph showing a mixotrophic microalgae culture volumetric growth rate (g/L/Day) correlated to concentration.

FIG. 35 is a graph showing a mixotrophic microalgae culture areal growth rate ($g/m^2$) correlated to concentration.

FIG. 36 is a graph showing a mixotrophic microalgae culture temperature over time.

FIG. 37 is a graph showing a mixotrophic microalgae culture pH over time.

FIG. 38 is a graph showing a mixotrophic microalgae culture dissolved oxygen concentration over time.

FIG. 39 is a graph showing a mixotrophic microalgae culture supplied photosynthetic active radiation over time.

FIG. 40 is a graph showing a mixotrophic microalgae culture nitrate concentration over time.

FIG. 41 is a graph showing the productivity in cell dry weight of mixotrophic microalgae cultures supplied with different organic carbon sources over time.

## DETAILED DESCRIPTION

### Introduction

The term "microorganism" refers to microscopic organisms such as microalgae and cyanobacteria. Microalgae include microscopic multi-cellular plants (e.g. duckweed), photosynthetic microorganisms, heterotrophic microorganisms, diatoms, dinoflagelattes, and unicellular algae.

Microorganisms that may grow in mixotrophic culture conditions comprise microalgae and cyanobacteria. Non-limiting examples of mixotrophic microorganisms may comprise organisms of the genera: *Agmenellum, Amphora, Anabaena, Anacystis, Apistonema, Arthrospira (Spirulina), Botryococcus, Brachiomonas, Chlamydomonas, Chlorella, Chloroccum, Cruciplacolithus, Cylindrotheca, Coenochloris, Cyanophora, Cyclotella, Dunaliella, Emiliania, Euglena, Extubocellulus, Fragilaria, Galdieria, Goniotrichium, Haematococcus, Halochlorella, Isochyrsis, Leptocylindrus, Micractinium, Melosira, Monodus, Nostoc, Nannochloris, Nannochloropsis, Navicula, Neospongiococcum, Nitzschia., Odontella, Ochromonas, Ochrosphaera, Pavlova, Picochlorum, Phaeodactylum, Pleurochyrsis, Porphyridium, Poteriochromonas, Prymnesium, Rhodomonas, Scenedesmus, Skeletonema, Spumella, Stauroneis, Stichococcus, Auxenochlorella, Cheatoceros, Neochloris, Ocromonas, Porphiridium, Synechococcus, Synechocystis, Tetraselmis, Thraustochytrids, Thalassiosira,* and species thereof.

Non-limiting examples of suitable microorganism species for mixotrophic growth using acetic acid as an organic carbon source may comprise organisms of the genera: *Chlorella, Anacystis, Synechococcus, Synechocystis, Neospongiococcum, Chlorococcum, Phaeodactylum, Spirulina, Micractinium, Haematococcus, Nannochloropsis, Brachiomonas,* and species thereof.

The organic carbon sources suitable for growing a microorganism mixotrophically or heterotrophically may comprise: acetate, acetic acid, ammonium linoleate, arabinose, arginine, aspartic acid, butyric acid, cellulose, citric acid, ethanol, fructose, fatty acids, galactose, glucose, glycerol, glycine, lactic acid, lactose, maleic acid, maltose, mannose, methanol, molasses, peptone, plant based hydrolyzate, proline, propionic acid, ribose, sacchrose, partial or complete hydrolysates of starch, sucrose, tartaric, TCA-cycle organic acids, thin stillage, urea, industrial waste solutions, yeast extract, and combinations thereof. The organic carbon source may comprise any single source, combination of sources, and dilutions of single sources or combinations of sources.

While microorganisms capable of mixotrophic growth may also grow in 100% phototrophic conditions or 100% heterotrophic conditions, it has been found that the combination of light and an organic carbon source may outperform phototrophic conditions in a bioreactor, including open systems. Microorganisms capable of mixotrophic growth may also grow in a combination of conditions, such as transitioning between phototrophic, mixotrophic, and heterotrophic conditions. Culturing methods with transitions between trophic conditions may regulate the feed source for bacteria, while allowing the mixotrophic microorganisms to continue to grow in the varied conditions.

For example, microalgae growth may slow down in phototrophic conditions compared to mixotrophic or heterotrophic conditions, but the bacteria growth may slow down as well and not grow phototrophically if the bacteria lacks a photosynthetic metabolism. The ability to grow mixotrophically in non-axenic conditions also provides a method simpler than traditional fermentation and closer to the simplicity of phototrophic methods. The optimization of productivity from a mixotrophic microorganism in non-axenic conditions therefore comprises the selection of an organic carbon source, the system and methods for administering the organic carbon source, the systems and methods for applying the optimal type of light and amount of light, the systems and methods for administering nutrients, the systems and methods for controlling the pH level, and the systems and methods for controlling the contaminating organisms population (e.g., bacteria, fungi). Using an acetic acid/pH auxostat system provides an efficient method for growing a mixotrophic culture of microorganisms in non-axenic conditions by maintaining the organic carbon source at a constant or substantially constant level in the culture while helping to maintain culture conditions that inhibit contaminating bacterial growth.

Abbreviations in a formula such as g/m$^2$ d; g/L d; h/day; L/d; Watts h/g; or mg/L d are used throughout this text. g/m$^2$ d means gram per meter squared per day or grams per meter squared per day. g/L d means gram per liter per day or grams per liter per day; L/d means liter per day or liters per day; h/day and h/d mean hours per day; Wh/g means Watt hour per gram or Watt hours per gram.

The term "productivity" refers to the measure of the microalgae or cyanobacteria growth rate.

The term "areal productivity" or "areal growth rate" (sometimes also spelled aerial) means the mass of microalgae or cyanobacteria produced per unit land area per day. An example of such rate is grams per square meter per day (g/m$^2$ d) which is the grams of microalgae or cyanobacteria produced per m$^2$ of the reactor area per day.

The term "volume productivity" or "volumetric growth rate" means the mass of microalgae or cyanobacteria produced per unit culture volume per day. An example of such a unit is g/L d (grams per liter per day) which is the grams of microalgae or cyanobacteria produced in each liter of the culture per day. Example 4 refers to a pond with 10 cm depth, hence the aerial productivity g/m$^2$/day is 1/100 times that of the volumetric productivity g/L/day.

An auxostat is a device that uses the rate of feeding to control a state variable in a continuous culture. The organisms in the culture establish their own dilution rate. An auxostat tends to be much more stable at high dilution rates than a chemostat commonly known in the art. Population selection pressures in an auxostat lead to cultures that grow rapidly. Practical applications include high-rate propagation, destruction of wastes with control at a concentration for maximum rate, open culturing because potential contaminating organisms cannot adapt before washing out, and operation of processes that benefit from careful balance of the ratios of nutrient concentrations.

The term "pH auxostat" refers to the microbial cultivation technique that couples the addition of fresh medium (e.g., medium containing organic carbon or acetic acid) to pH control. As the pH drifts from a given set point, fresh medium is added to bring the pH back to the set point. The rate of pH change is often an excellent indication of growth and meets the requirements as a growth-dependent parameter. The feed will keep the residual nutrient concentration in balance with the buffering capacity of the medium. The pH set point may be changed depending on the microorganisms present in the culture at the time. The microorganisms present may be driven by the location and season where the bioreactor is operated and how close the cultures are positioned to other contamination sources (e.g., other farms, agriculture, ocean, lake, river, waste water). The rate of medium addition is determined by the buffering capacity and the feed concentration of the limiting nutrient and not directly by the set point (pH) as in a traditional auxostat. The pH auxostat is robust but controls nutrient concentration indirectly. The pH level represents the summation of the production of different ionic species and ion release during carbon and nutrient uptake. Therefore the pH level can move either up or down as a function of growth of the microorganisms. The most common situation is pH depression caused by organic acid production and ammonium uptake. However, for microorganisms growing on protein or amino acid-rich media, the pH level will rise with growth because of the release of excess ammonia.

The terms "microbiological culture", "microbial culture", or "microorganism culture" refer to a method or system for multiplying microorganisms through reproduction in a predetermined culture medium, including under controlled laboratory conditions. Microbiological cultures, microbial cultures, and microorganism cultures are used to multiply the organism, to determine the type of organism, or the abundance of the organism in the sample being tested. In liquid culture medium, the term microbiological, microbial, or microorganism culture generally refers to the entire liquid medium and the microorganisms in the liquid medium regardless of the vessel in which the culture resides. A liquid medium is often referred to as "media", "culture medium", or "culture media". The act of culturing is generally referred to as "culturing microorganisms" when emphasis is on plural microorganisms. The act of culturing is generally referred to as "culturing a microorganism" when importance is placed on a species or genus of microorganism. Microorganism culture is used synonymously with culture of microorganisms.

The terms "monoalgal" and "unialgal" refer to a microalgae or cyanobacteria culture that is operated under non-axenic conditions but dominated by a single microalgae or cyanobacteria genus or species. In spite of the presence of other heterotrophic microorganisms (i.e., non-axenic conditions), the culture remains stable due to the inorganic mineral culture medium used in cultures. Monoalgal cultures might be employed for heterotrophic cultures but the combination of an organic medium with the presence of heterotrophic bacteria and fungi does not warrant the stability of the monoalgal culture found in phototrophic conditions *"per se"* and therefore the term is less appropriate. Monoalgal culture may also be used for defining a non-axenic microorganism culture dominated by a single genus or species.

The term "inoculate" refers to implanting or introducing microorganisms into a culture medium. Inoculate or inoculating a culture of microorganisms in the described culture conditions throughout the specification refers to starting a culture of microorganisms in the culture conditions, as is commonly used in the art of microorganism culturing. The microorganisms that are introduced into a culture medium may be referred to as seed or inoculum.

The term "ozone" means a form of oxygen, $O_3$, with a peculiar odor suggesting that of weak chlorine, produced when an electric spark or ultraviolet light is passed through air or oxygen. Ozone is a colorless unstable toxic gas with powerful oxidizing properties, formed from oxygen by electrical discharges.

The term "coagulate" means to cause transformation of a liquid suspension into a viscous or thickened soft, semisolid, or solid mass. Coagulate means to dewater, as in to remove enough water to cause a liquid suspension to become a thickened soft, viscous semisolid, or solid mass. Dewatering methods may be used in conjunction with microorganism cultures to cause the microorganisms to coagulate and form a denser mass that may be more suitable for harvesting and downstream processing.

The terms "mixotrophic" and "mixotrophy" refer to culture conditions in which light, organic carbon, and inorganic carbon (e.g., carbon dioxide, carbonate, bi-carbonate) may be applied to a culture of microorganisms. Microorganisms capable of growing in mixotrophic conditions have the metabolic profile of both phototrophic and heterotrophic microorganisms, and may use both light and organic carbon as energy sources, as well as both inorganic carbon and organic carbon as carbon sources. A mixotrophic microorganism may be using light, inorganic carbon, and organic carbon through the phototrophic and heterotrophic metabolisms simultaneously or may switch between the utilization of each metabolism. A microorganism in mixotrophic culture conditions may be a net oxygen or carbon dioxide producer depending on the energy source and carbon source utilized by the microorganism. Microorganisms capable of mixotrophic growth comprise microorganisms with the natural metabolism and ability to grow in mixotrophic conditions, as well as microorganisms which obtain the metabolism and ability through modification of cells by way of methods such as mutagenesis or genetic engineering.

The terms "phototrophic", "phototrophy", "photoautotrophy", "photoautotrophic", and "autotroph" refer to culture conditions in which light and inorganic carbon (e.g., carbon dioxide, carbonate, bi-carbonate) may be applied to a culture of microorganisms. Microorganisms capable of growing in phototrophic conditions may use light as an energy source and inorganic carbon (e.g., carbon dioxide) as a carbon source. A microorganism in phototrophic conditions may produce oxygen.

The terms "heterotrophic" and "heterotrophy" refer to culture conditions in which organic carbon may be applied to a culture of microorganisms in the absence of light. Microorganisms capable of growing in heterotrophic conditions may use organic carbon as both an energy source and as a carbon source. A microorganism in heterotrophic conditions may produce carbon dioxide.

The term "axenic" describes a culture of an organism that is entirely free of all other "contaminating" organisms (i.e., organisms that are detrimental to the health of the microalgae or cyanobacteria culture). Throughout the specification, axenic refers to a culture that when inoculated in an agar plate with bacterial basal medium, does not form any colonies other than the microorganism of interest. Axenic describes cultures not contaminated by or associated with any other living organisms such as but not limited to bacteria, cyanobacteria, microalgae and/or fungi. Axenic is usually used in reference to pure cultures of microorganisms that are completely free of the presence of other different organisms. An axenic culture of microalgae or cyanobacteria is completely free from other different organisms.

The term "harvesting" refers to removing the culture of microorganisms from the culturing vessel and/or separating the microorganisms from the culture medium. Harvesting of microorganisms may be conducted by any method known in the art such as, but not limited to, skimming, draining, dissolved gas flotation, foam fractionation, centrifugation, filtration, sedimentation, chemical flocculation, and electro-dewatering.

Methods of mixotrophic culturing

A method of culturing microorganisms in non-axenic mixotrophic conditions comprises: inoculating an aqueous culture medium with a culture of microorganisms comprising at least some contaminating bacteria in a culturing vessel; supplying the culture of microorganisms with at least some light; and supplying an organic carbon source to the culture of microorganisms. The selection of an organic acid as the organic carbon source may contribute to maintaining a level of contaminating bacteria below an acceptable threshold to achieve high growth rates. It is recognized that in practice, a culture comprising microorganisms in non-axenic conditions such as an illuminated culture of microalgae or cyanobacteria with an organic carbon source will have at least some contamination bacteria, as the practical ability to reduce the bacteria population close to zero (e.g., by steam sterilization or other known procedures) may be cost prohibitive for large volume illuminated cultures with a culture density of 0.05-10 g/L. Additionally, it may not be desirable to entirely eliminate the bacteria population of a culture, as certain bacteria may be involved in the production of essential nutrients for microalgae or cyanobacteria, such as cyanocobalamin, or other valuable products. Therefore, the level of contaminating bacteria may be maintained below 25%, 20%, 10%, or 5% of the total cell counts of the culture.

As previously described, maintaining a certain level of specific types of bacteria may be beneficial to a culture of microalgae or cyanobacteria. Bacteria that may be present in cultures of microalgae and cyanobacteria comprise, but are not limited to: *Achromobacter* sp., *Acidovorax* sp., *Acinetobacter* sp., *Aeromonas* sp., *Agrobacterium* sp., *Alteromonas* sp., *Ancylobacter* sp., *Aquaspirillum* sp., *Azospirillum* sp., *Azotobacter* sp., *Bacillus* sp., *Bergeyella* sp., *Brevundimonas* sp., *Brochothrix* sp., *Brumimicrobium* sp., *Burkholderia* sp., *Caulobacter* sp., *Cellulomonas* sp., *Chryseobacterium* sp., *Curtobacterium* sp., *Delftia* sp., *Empedobacter* sp., *Enterobacter* sp., *Escherichia* sp., *Flavobacterium* sp., *Gemmatimonas* sp., *Halomonas* sp., *Hydrogenophaga* sp., *Janthinobacterium* sp., *Lactobacillus* sp., *Marinobacter* sp., *Massilia* sp., *Microbacterium* sp., *Myroides* sp., *Pantoea sp.,* *Paracoccus* sp., *Pedobacter* sp., *Phaeobacter* sp., *Phyllobacterium* sp., *Pseudoalteromonas* sp., *Pseudomonas* sp., *Rahnella* sp., *Ralstonia* sp., *Rhizobium* sp., *Rhodococcus* sp., *Roseomonas* sp., *Sphingobacterium* sp., *Sphingomoas* sp., *Staphylococcus* sp., *Stenotrophomonas* sp., *Vibrio* sp., and *Zobelliae* sp.

Bacteria that have a negative or harmful effect on the microalgae and cyanobacteria may be designated as contaminating bacteria. The bacteria that may have a negative or harmful effect on microalgae or cyanobacteria in a culture comprise, but are not limited to: *Achromobacter* sp., *Acidovorax* sp., *Aeromonas* sp., *Agrobacterium* sp., *Alteromonas* sp., *Aquaspirillum* sp., *Azospirillum* sp., *Azotobacter* sp., *Bergeyella* sp., *Brochothrix* sp., *Brumimicrobium* sp., *Burkholderia* sp., *Caulobacter* sp., *Cellulomonas* sp., *Chryseobacterium* sp., *Curtobacterium* sp., *Delftia* sp., *Empedobacter* sp., *Enterobacter* sp., *Escherichia* sp., *Flavobacterium* sp., *Marinobacter* sp., *Microbacterium* sp., *Myroides* sp., *Paracoccus* sp., *Pedobacter* sp., *Phaeobacter* sp., *Pseudoalteromonas* sp., *Pseudomonas* sp., *Rahnella* sp., *Ralstonia* sp., *Rhizobium* sp., *Rhodococcus* sp., *Roseomonas* sp., *Staphylococcus* sp., *Stenotrophomonas* sp., *Vibrio* sp., *Zobelliae* sp. and other bacteria which share similar characteristics.

The bacteria that may have a neutral or beneficial effect on microalgae or cyanobacteria in a culture comprise, but are not limited to: *Acidovorax* sp., *Acinetobacter* sp., *Aeromonas* sp., *Agrobacterium* sp., *Alteromonas* sp., *Ancylobacter* sp., *Azospirillum* sp., *Azotobacter* sp., *Bacillus* sp., *Brevundimonas* sp., *Brumimicrobium* sp., *Burkholderia* sp., *Caulobacter* sp., *Cellulomonas* sp., *Delftia* sp., *Empedobacter* sp., *Gemmatimonas* sp., *Halomonas* sp., *Hydrogenophaga* sp., *Janthinobacterium* sp., *Lactobacillus* sp., *Marinobacter* sp., *Pantoea sp.,* *Paracoccus* sp., *Phaeobacter* sp., *Phyllobacterium* sp., *Pseudoalteromonas* sp., *Pseudomonas* sp., *Rhizobium* sp., *Sphingomoas* sp., *Zobelliae* sp. and other bacteria which share similar characteristics. While bacteria in a particular genus generally have the same characteristics, it is recognized that a genus of bacteria with the majority of species generally identified as harmful to microalgae or cyanobacteria may also include a particular species within the genus which is neutral or beneficial to a specific culture of microalgae or cyanobacteria, and vice versa. For example, many species of *Pseudomonas* have been observed to be harmful to microalgae, however literature has described certain species of *Pseudomonas* with anti-fungal functionality which may be beneficial to a culture of microalgae or cyanobacteria.

Bacteria that provide a neutral or beneficial effect may be added to a culture of microalgae or cyanobacteria in a probiotic role to help control the level of contaminating bacteria, increase growth yield of the microalgae or cyanobacteria, or increase culture longevity. In one non-limiting example, recent data has shown by inoculating mixotrophic *Chlorella* cultures with *Bacillus* sp., *Rhizobium* sp., and *Sphingomonas* sp., the *Chlorella* grew well at 35°C and outperformed the control cultures that were not inoculated with bacteria. Other additives may be applied to the media that inhibit gram (+) and/or gram (-) bacteria to reduce growth of all bacteria and/or specific bacteria present in the culture.

Some bacteria species that have been observed to have a neutral or beneficial effect on microalgae or cyanobacteria and may be added to the culture in a probiotic role to provide functions such as: cycle organic and inorganic nutrients; produce valuable industrial and pharmaceutical products such as extracellular polymer substances, nutrients, vitamins, and chelated minerals; produce antifungal agents; produce growth enhancers; produce antibiotics; produce biocompounds; fix nitrogen; transform nutrients; decompose organic matter; assist in maintaining a balance of biological equilibrium in the microorganism culture; and assist in nitrification and denitrification. Some bacteria may have photopigments that react to light similar to microalgae or cyanobacteria. As one non-limiting example, *Azospirillum* sp. grown in a culture with *Chlorella* may increase pigment content, lipid content, lipid variety, and growth. *Sphingomonas* sp. is known in the literature to be associated with plants and able to fix nitrogen. Bacteria with photopigments may also be manipulated with light intensity and/or specific light wavelengths to influence a culture.

The microorganisms are inoculated in an aqueous culture medium contained in any suitable vessel for growth. The culture medium may be any liquid culture medium suitable for culturing microorganisms, such as but not limited to a BG-11 culture medium, a modified BG-11 culture medium, an f/2 culture medium, and a modified f/2 culture medium. The culture medium may comprise any one or more of: ocean water, lake water, river water, wastewater, or other available water sources; available water sources cleaned via filtration or sterilization before inoculation with a microorganism culture; and an aqueous media inoculated with beneficial microbes (e.g., bacteria) to jumpstart the microorganism culture. Parameters of a culture may be manipulated and beneficial microbes (e.g., bacteria) may be added to the culture media as needed depending on microorganisms present in the culture and health of the culture. The culturing vessel may comprise a tank, bioreactor, photobioreactor, pond, raceway pond, tubular reactor, flat panel reactor, trough, column bioreactor, bag bioreactor, or any other bioreactor known in the art. The vessel may be open. The vessel may be closed. The vessel may be located indoors. The vessel may be located outdoors. The vessel may be located outside but disposed within a greenhouse, structure, or cover that substantially encloses the reactor to minimize ingress of unwanted elements and foreign materials from the environment, and blocks at least some light or wavelengths of light.

The microorganism culture may be maintained at a constant or substantially constant pH level and temperature. The microorganism culture may be operated between a range of pH levels or a hysteresis range of temperatures, including temperatures that follow a diurnal cycle, or a range of pH levels and temperatures. The pH level ranges from about 6 to 9 and the temperature ranges from about 10°C to 30°C. The pH level ranges from about 1 to about 5 and the temperature ranges from 30°C to 50°C. The pH level and temperature may be maintained with the identified ranges for at least a portion of the growth cycle which may correlate with the daylight hours or hours after sunset. The pH level may be about 7.5 and the temperature may be about 25-28 °C.

The temperature of the culture may be controlled with a heat exchanger such as, but not limited to, cooling or heating coils. The pH set point may be changed within the range suitable for microalgae growth during the culturing depending on the composition of microorganisms present. A change in pH may be used to shock (i.e., stress) bacteria and reduce proliferation of contaminating bacteria in the culture harmful to the primary microalgae or cyanobacteria. A change in pH may be combined with a cessation of the carbon source supply for a period of time (e.g., 4 hours - 48 hours), manipulation of dissolved oxygen (DO) levels, modification of temperature, addition of bactericidal agents, addition of amino acids or other feed sources, and combinations thereof that may reduce the proliferation of bacteria that may be harmful to the microalgae or cyanobacteria and/or promote the proliferation of bacteria that may be beneficial/non-harmful bacteria.

An illumination source comprising photosynthetically active radiation (PAR) supplies the culture with at least some light for photosynthetic activity. The source of light may be natural, artificial, or any combination thereof. The period of light exposure (i.e., photoperiod) may range from about 0 to 24 h/day. The period of light exposure (i.e., photoperiod) may range from about 10 to 16 h/day. The supply of light may be continuous, discontinuous (e.g., flashing), constant intensity, or variable intensity. The natural light source may comprise solar radiation. Alternately, the culture may be exposed to light on an intermittent basis, at the end of the culture life, for a few minutes per day, or for one day and the like. The artificial light source may comprise light emitting diodes (LEDs), micro LEDs, fluorescent lights, incandescent lights, gas lamps, or halogen lamps.

The natural light source may be filtered or the artificial light source may be tuned to limit the supplied light to a specific wavelength spectrum or combination of specific wavelength spectrums such as, but not limited, violet (about 380-450 nm), blue (about 450-495 nm), green (about 495-570 nm), yellow (570-590 nm), orange (about 590-620 nm), red (about 620-750 nm), and far red (about 700-800 nm) light spectrums. LEDs tuned to a specific wavelength spectrum or light wavelengths filtered by specific greenhouse films may be used to manipulate growth and product production of the microorganisms in the culture. Finishing steps with LEDs of a specific wavelength may be used prior to, during, or after harvest to influence the product profile in the microorganisms. Different intensities and/or wavelengths of light may be applied to microorganisms at particular times to: enhance growth, enhance product formation, manipulate pigment formation, or "naturally sterilize" the culture with ultraviolet (UV) light. For example, increasing the intensity of UV light in a *Haematococcus* culture may yield cyst and pigment formation.

The culture may be mixed by hydraulic mixing (e.g., pumps), mechanical mixing (e.g., agitators, stirrers, thrusters), or paddle wheels. The culture may be aerated with air, carbon dioxide, oxygen, or any other suitable gas. The aeration may be provided by a gas injector, porous diffuser, micropore diffuser, gas permeable membranes, microbubble generator, venturi injection, or a microbubble fluidic oscillator. Mixing, agitation, and/or aeration of the culture allows circulation of the microorganisms in the culture for an even distribution of nutrients, gases, and the organic carbon source, as well as access to light.

An organic acid such as acetic acid may be used as an organic carbon source and supplied to the microorganism culture from a feed tank through a pH auxostat system. The pH auxostat system may comprise a solenoid valve, a peristaltic pump, a pH probe and a pH controller. The pH auxostat system may comprise a drip application device controlled by a needle valve, a metering pump or a peristaltic pump, and a pH controller. Carbon and nitrogen uptake (i.e., sodium acetate, sodium nitrate) and photosynthetic activity (i.e., sodium bicarbonate uptake) cause the pH level of the culture to rise. The pH controller may be set at a threshold level (i.e., set point) and activate the auxostat system to supply acetic acid to the culture when the measured pH level is above the set threshold level. The frequency of pH measurements, administration of acetic acid by the auxostat system, and mixing of the culture are controlled in combination to keep the pH value substantially constant. The acetic acid feed may be diluted in water to a concentration below 100% and as low as 0.5%, with a preferable concentration between 15% and 50%. The acetic acid may be at concentrations below 10% in order to continuously dilute the culture of microorganisms. The acetic acid may be mixed together with other media or organic carbon sources.

The organic carbon source, such as acetic acid, may be combined or mixed together with other nutrient components such as nitrogen or phosphorus sources. The other nutrients may be added directly to the organic carbon source, such as acetic acid, and a single solution (or multiple solutions) may be added to the culture via the acetic acid supply controlled by the pH auxostat. The nutrients may be added directly to the acetic acid or alternate organic carbon source prior to supplying the solution to the culture. When using an organic acid, the need for filtration of the nutrient media may be reduced because the organic acid, such as acetic acid, keeps the nutrient media sterile. The amount of organic carbon source, such as acetic acid, consumption may be monitored by measuring the level of the organic carbon (e.g., acetic acid) in the feed tank, which may be correlated with microorganism cell growth. Nitrates (e.g., $NO_3$), phosphates, and other nutrient known to be used by plants (e.g., a non-limiting set of nutrients would be iron, cobalt, copper, sodium, manganese, zinc, molybdenum, silica, salts, and combinations thereof) may also be added to the culture to maintain nitrate and nutrients at a desired level. The organic carbon source and at least one other nutrient may be in a concentrated form. The organic carbon source and at least one other nutrient may be in a diluted form.

The contamination level in the culture of mixotrophic microorganisms (e.g., microalgae or cyanobacteria) may be managed to limit the residual or free floating feed sources available to the contaminating microorganisms (e.g., bacteria, fungi). The organic carbon feed and at least one other nutrient feed maybe mixed and supplied together to introduce the organic carbon and at least one other nutrient together in an amount that will be substantially consumed by the mixotrophic microorganisms and minimize the amount of residual or free floating organic carbon and the at least one other nutrient available for contaminating microorganisms. This may effectively maintain the culture concentration at near zero for the organic carbon and at least one other nutrient. The ratio of organic carbon to the at least one other nutrient may be selected so that the mixotrophic microorganism consumption of the organic carbon matches the consumption of the at least one other nutrient. The consumption rates of organic carbon and nutrients for various microorganisms separately may be determined through experimentation and review of available literature.

The organic carbon source comprises acetic acid. The acetic acid may be diluted to a concentration of about 30% or less. In some embodiments, the at least one other nutrient comprises $NO_3$. In some embodiments, the ratio of $NO_3$:Acetic Acid may range from 0.5:10 to 2:10, preferably about 1:10. Acetic acid may comprise acetic acid and its precursors, such as acetate and acetic anhydride.

The ratio of organic carbon to the at least one other nutrient may be selected so that the dosage of the at least one other nutrient spikes the concentration in the culture medium to maintain a baseline level. One non-limiting application may be in culturing a wastewater treatment microorganism such as, not limited to, bacteria, for purposes of waste remediation in a wastewater culture medium. The organic carbon may be mixed and administered with another growth limiting nutrient, such as nitrates, to effectively maintain the culture concentration of nitrates at a minimum level or baseline such as, but not limited to, 100 ppm.

Mixotrophic microorganism growth consumes and produces oxygen thus altering the dissolved oxygen (DO) concentration (mg/L) in the culture medium. Dissolved oxygen concentration may be controlled to boost mixotrophic microorganism growth and keep a contaminating bacterial population controlled. Cellular respiration in microalgae and cyanobacteria seems to be less efficient than in bacteria, which can scavenge oxygen at low concentration or even grow anaerobically better than microalgae and cyanobacteria. Therefore dissolved oxygen may be utilized as a variable parameter to manage the contaminating bacterial populations in mixotrophic cultures with little to no effect on microalgae and cyanobacteria productivity and viability. Oxygen transfer may be reduced or increased to manage the contaminating bacterial populations within the mixotrophic cultures to increase culture longevity and reduce bacterial contamination.

The dissolved oxygen concentrations in the culture solution may be controlled mechanically, chemically or biologically. Mechanical control may comprise pure air injection; blended air with increased oxygen concentrations by using an oxygen concentrator or compressed oxygen injection; or blended air with nitrogen which reduces dissolved oxygen concentrations. Mechanical control may also comprise the design and dimensioning of the reactor, depth of the culture unit, mixing rates, and surface area of the reactor which dictates air/water gas exchange. Chemical control may comprise sodium sulfite which reduces dissolved oxygen concentrations, or other chemicals that would increase dissolved oxygen, such as ozone. Sodium sulfite may act as an oxygen scavenger, for example two molecules of sodium sulfite will react with two atoms of oxygen when dissolved in water. Therefore to remove 1 ppm of oxygen, 7.8 ppm of sodium sulfite may be utilized ($2Na + 2SO_3 + 2O = 2Na + 2SO_4$).

A non-limiting list of oxygen scavengers includes sodium sulfite and hydrazine, (Sigma-Aldrich St. Louis, MO), Eliminox® carbohydrazide and SurGard® erythrobate (Nalco Chemical Co. Naperville, IL), Mekor® methylethylketoxime (*Drew* Chemical Corporation, Boonton, NJ), Magni-Form® hydroquinone (Betz Laboratories, Trevose, PA), Steamate® diethylhydroxylamine (Dearborn Chemical Co. Lake Zurick, IL). The dissolved oxygen may also be controlled biologically by transitioning the culture between mixotrophic and phototrophic conditions. When dissolved oxygen concentrations reach the targeted concentrations and contaminating bacteria populations have been reduced, the system may be transitioned from phototrophic conditions back to mixotrophic conditions thus creating a cyclic pattern that reduces contaminating bacteria and increases microalgae or cyanobacteria culture longevity.

Threshold levels (i.e., set points) for dissolved oxygen in a mixotrophic microorganism culture may range from about 0.1 mg $O_2$/L to about 30 mg $O_2$/L depending on bacterial population and species, as well as microalgae or cyanobacteria population and species. Dissolved oxygen ranges may be held at target concentrations for a sustained period of time. When contaminating bacteria populations reach concentrations unsuitable for longevity and viability of the mixotrophic microorganism culture, the dissolved oxygen may be increased to target concentrations which reduce contaminating bacteria populations without affecting the microalgae or cyanobacteria culture viability. Target concentrations of dissolved oxygen may be between 1-6 mg $O_2$/L, or above atmospheric saturation concentrations such as 100 to 300 % saturation. The organic carbon source may be supplied to the culture until a measured dissolved oxygen level of the culture reaches a critical level below about 2 mg $O_2$/L.

A factor contributing to the efficiency of the pH auxostat system to supply acetic acid (i.e., organic carbon) to the culture may include the ability to initially activate the auxostat system and start the supply of acetic acid to control the pH level. The acetic acid/pH auxostat system may be initially activated by the photosynthetic activity of the microorganisms raising the pH of the culture. Sodium acetate, sodium hydroxide, or potassium hydroxide may be added to the initial culture medium to increase the residual acetic acid concentration and automatically activate the acetic acid/pH auxostat system prior to the start of photosynthetic activity by the microorganisms. 0.05-10 g/L of sodium acetate is initially added to the culture medium. In further embodiments, 0.1-6 g/L of sodium acetate may be initially added to the culture medium to assist with the transition from phototrophic to mixotrophic conditions. The sodium acetate concentration may be above 1.6 g/L in order to inhibit the growth of contaminating microorganisms (e.g., bacteria, fungi). The concentration of sodium acetate may be supplied for at least the first day of microorganism growth. In further embodiments, the sodium acetate may be supplied for the first 1-5 days of microorganism growth, and preferably the first two days of microorganism growth.

Alternatively, sodium acetate may be added to the nutrient formulation of the culture medium to ensure that the initial concentration of sodium acetate is present. The culture medium with the nutrient formulation comprising sodium acetate may also be added continually to the culture as harvesting takes place. The organic carbon source may be added in low levels to the make-up water used to refill the culture and the water used to flush the culturing system as an alternative to a system which doses the culture with an organic carbon source.

Once the microorganism culture reaches a desired concentration or maturity, at least a portion of the microorganism culture may be harvested for further processing. The microorganisms may be harvested by any method known in the art such as, but not limited to, dissolved gas flotation, foam fractionation, centrifugation, filtration, sedimentation, chemical flocculation, and electro-dewatering. The harvesting may take place continuously or in a batch method that occurs multiple times a day, daily, after a certain number of days, or weekly.

An ammonia auxostat or other pH changing media may also be used. The addition of organic carbon may be balanced with carbon dioxide to cycle the organic matter within the culture and allow the contaminating bacteria to be controlled or kept in check without dominating a culture (as defined by greater than 50% of living cells in the culture comprising contaminating bacteria). Manipulating the carbon source supply (e.g., acetic acid or $CO_2$) may allow for control of the pH level, but the swings in pH level may be intentionally made larger to maintain the balance between dominance of microalgae or cyanobacteria over the contaminating bacteria in the culture (e.g., pH level swings from 7.5 to 8.5, or from 6.5 to 9 or any range with at least a 0.5 pH difference within the culture). Such manipulations of the pH level may affect the contaminating bacteria to a greater degree than the microalgae or cyanobacteria because the pH levels are within the range of growth for microalgae or cyanobacteria. The pH level may be maintained in a defined hysteresis range that inhibits the proliferation of contaminating organisms in the microorganism culture.

Contamination control methods

The use of an organic carbon source in the culture medium introduces a higher risk of bacterial contamination of the microorganism culture than in a phototrophic culture medium without an organic carbon source. With some species of bacteria being able to grow faster than microalgae or cyanobacteria, the bacteria may overtake the microalgae or cyanobacteria culture resources and the microalgae or cyanobacteria themselves. Therefore, the ability to control bacterial contamination is one factor contributing to the efficiency of a mixotrophic culture. An organic acid such as acetic acid has been found to inhibit bacterial growth in certain conditions, which may be associated with the denaturation of the enzyme responsible for the synthesis of methionine (o-succinyltransferase). Bacterial proliferation has been found to be faster in a glucose-containing culture medium than in an acetic acid containing culture medium, demonstrating the benefit of selecting acetic acid as the organic carbon source. Additionally, acetic acid was found to further decrease the bacterial resistance to oxidative stress (i.e., ozone, hydrogen peroxide) than was observed with glucose fed cultures.

Keeping the pH above 7.5 and temperature below 30°C in a minimal mineral defined medium specific for a genus of microalgae or cyanobacteria are conditions that have been shown to be sub-optimal conditions for the proliferation of contaminating organisms such as bacteria. The pH level may be below 5 and temperature between 30°C to 50°C. Through contamination control methods described herein, including the utilization of the acetic acid/pH auxostat system for administering acetic acid to the non-axenic culture, the contamination bacterial cell counts of the culture may be maintained below 25% of the total, below 20% of the total cells, below 10% of the total cells, and preferably below 5% of the total cells of the culture (<0.05% total biomass) through a culturing method that may comprise a combination of residual acetic acid in the culture medium, the maintenance of a constant pH level, or use of an oxidative agent. A heat exchanger which keeps the temperature constant, such as cooling or heating coils, also improves the control over the contaminating bacteria population when used in combination with other contamination control methods, such as but not limited to the acetic acid/pH auxostat system and oxidative agents.

Additional methods of contaminating bacteria and organism control in a mixotrophic culture may comprise the application of hydrogen peroxide, ozone, antibiotics, ultraviolet (UV) radiation/sterilization, or other oxidizing agents (e.g., chlorine, chlorite, chlorate, hypochlorite, nitric acid, chromium, permanganate, silver oxide, bromine). The methods of controlling contamination in the mixotrophic culture may be used individually or in combination. Adding hydrogen peroxide to a culture medium comprising contaminating bacteria has been shown to inhibit the growth of the contaminating bacteria at applications between 2.5 and 30 mM $H_2O_2$. Bacteria growing in mixotrophic culture mediums comprising acetic acid have been shown to be more susceptible to oxidative stress than in mixotrophic culture mediums comprising glucose. Ozone may be applied to a culture through any known gas injection method such as, but not limited to, sparging and venturi injection. Ozone treatments may be applied to the microorganism culture at concentrations of 0.01-2.0 mg/L, and preferably at concentrations of 0.01-0.50 mg/L. Antibiotic treatments may comprise, but are not limited to, penicillin (100-500 mg/L), tetracycline (10-100 mg/L), chloramphenicol (1-20 mg/L), and aureomycin (1-20 mg/L).

The use of electro-coagulation for periodic concentration and purging of a culture provides an example for a method comprising the daily or continuous harvesting of a culture to contribute to controlling contamination through the regular concentration and removal of the microalgae or cyanobacteria from contaminated media, and replacement of the culture in treated or new media. A method of harvesting and purging a culture for contamination control may also be performed through known methods of harvesting or separating the microalgae from a culture such as, but not limited to, foam fractionation, dissolved gas flotation, centrifugation, and flocculation. When a harvesting and purging method is used, the addition of the organic carbon source may need to be adjusted accordingly. The harvesting and purging may be promoting the health of a microalgae or cyanobacteria population and therefore its resistance towards contamination. The separated culture medium may be processed to convert the decaying organic matter (mineralization) into an available carbon source for the mixotrophic microorganisms.

Alternatively, sonication may be used to control the contamination in a mixotrophic culture. The culture may be subjected to sonic energy at various intensities to reduce contamination. Sonication may reduce the contamination within the culture by creating gas bubbles the same size as the gas vacuoles inside the contaminating bacteria. As the gas bubbles burst, the same sized bubbles in the bacteria will resonate and burst as well. Sonication may also reduce the contamination within the culture by disrupting the cell walls of the contamination bacteria, which are weaker relative to the cell walls of microalgae, cyanobacteria, or diatoms. The sonic energy may be provided at about 40-99% intensity from a 30 kHz horn. The culture being treated with sonication may be cooled to maintain the temperature of the culture within a desired range. The sonication treatment may be used with a culture to raise the temperature and treat contamination simultaneously. Sonication may be used as a pre-treatment of a culture in combination with a cell wall weakening chemical or enzyme. The sonication horn may be in line with a flow path of the culture. The cells broken by sonication may be removed from the culture using any device known in the art such as, but not limited to, a dissolved gas flotation device, a foam fractionation device, or a protein skimmer.

Alternately, the addition of plant extracts to the culture may control contaminating bacteria by slowing growth of the bacteria to allow the microalgae or cyanobacteria to outcompete the contaminating bacteria within the culture. Cultures with contaminating bacteria may have reduced populations with a 1, 2, or 3 or more log reduction in population with a means described earlier.

Dissolved oxygen levels

Dissolved oxygen (DO) levels have been shown to be a limiting nutrient for optimal mixotrophic growth. Therefore, the ability to transfer gases, such as oxygen, to the culture at a high rate is one factor contributing to the efficiency of a mixotrophic culture. At steady state the oxygen transfer rate is equal to the rate of oxygen consumption by the microalgae or cyanobacteria cells. The gas-liquid interfacial mass transfer can be calculated using the following formula:

$$k_L a \cdot (C^* - C_L) = OUR$$

Hence $k_L a$= oxygen utilization rate/ (concentration gradient);
$k_L$ is the mass transfer coefficient for oxygen transfer into the liquid media;
$a$ is the interfacial surface area of gas per volume of liquid;

$k_L a$ is a metric of gas-liquid interfacial mass transfer measured in Hz or ($s^{-1}$), where larger values of $k_L a$ are equivalent to better mass transfer and
higher reactor performance as determined by the volumetric growth rate of microalgae.

By increasing the $k_L a$ in a vessel the limitations on growth of the microorganism due to insufficient supply of oxygen may be overcome. When culturing microalgae or cyanobacteria with acetic acid as the carbon source, the $k_L a$ may range from 2.00 x $10^{-3}$ $s^{-1}$ to 2.10 x$10^4$ $s^{-1}$. The $k_L a$ may be at least 2.40 x $10^{-3}$ $s^{-1}$. Methods for increasing the $k_L a$ in a vessel include decreasing the size of the gas bubbles and increasing the residence time, and may be achieved by: increasing the shear stress of the mechanical mixing, decreasing the size of injection points and/or increasing the number of injection points to dissolve more gas into the microorganism culture solution, or increasing the gas pressure. The increase in $k_L a$ may be achieved through any known system for injection of micro-stream of gas or micro-bubbles of gas into a liquid such as, but not limited to, a gas injector, a porous diffuser, a micropore diffuser, a gas permeable membrane, a microbubble generator, venturi injection, and a microbubble fluidic oscillator. An increase in gas pressure may be achieved by any known method such as, but not limited to, increasing the liquid column height. The oxygen transfer may also be improved by breaking the bubbles with mechanical shear and increasing the residence time of the bubbles through horizontal, vertical, or angular mixing. Increasing the photosynthetic contribution to mixotrophy will further improve the oxygen transfer through a chemical transfer path.

The acetic acid supply to the mixotrophic culture may be controlled by pH stabilization, resulting in the next limiting reactants for the microorganism culturing process being oxygen followed by nitrates and other nutrients. The nitrate, as well as other nutrients, may be on an automatic feeding regime based on feedback controls using sensors in the microalgae culture. Alternatively, one may feed nutrients, organic carbon, or air using the feedback from a nitrate sensor, electrical conductivity readings in freshwater systems, or dissolved oxygen sensor. Additionally, the relationship between the oxygen limitation and mechanical design allows changes in the mechanical design to correspondingly alter the dissolved oxygen conditions of the culture. The oxygen demand based on stoichiometry is calculated in Example 11. The culture may run at much higher $k_L a$ than the stoichiometric minimum (calculated in Example 11 as the ratio of oxygen utilization rate to the equilibrium concentration (C*)) to ensure that the reaction is limited metabolically and not mass transfer limited.

Various methods known in the art for enhancing the culture $k_L a$ and dissolved oxygen conditions may be used with a mixotrophic microorganism culturing process. The methods known in the art may be categorized into mechanical methods and chemical methods. The method of enhancing the culture $k_L a$ and oxygen conditions may be a mechanical method or combination of mechanical methods. The mechanical methods may include, but are not limited to, the addition of oxygen rich air, venturi injection, eductors, weirs, and lowering the temperature of the culture. The method of enhancing the culture $k_L a$ and oxygen conditions may be a chemical method, or combination of chemical methods. The chemical methods may include the addition of high surface area bubbles with culture compatible products such as ozone, nano-sized hydrocarbon bubbles, vegetable oil, mineral oil, and nano-sized metallic oxygen carriers. Any of the above methods may be used individually or in combination to enhance the culture $k_L a$ and oxygen conditions, including combinations of mechanical and chemical methods. Of these methods, the mechanical methods may be the least invasive to the culture growth, and potentially modular to deploy.

Additionally, dissolved oxygen concentration may be also be monitored to manage the contaminating bacterial population. Contaminating bacteria that grow aerobically may use the excess oxygen in the culture; therefore if the oxygen available to the contaminating bacteria is limited, then the growth of the contaminating bacteria may be limited.

Example 1

*Chlorella sp. SNL 333* (a local strain isolated by Arizona State University and initially reported as *Chlorella sp.* at the time of testing; further analysis was performed by Dr. Barbara Melkonian at the University of Cologne, Zülpicher Strasse 47 b, 50674 Köln, Germany, confirming the microalgae strain as sharing identifying characteristics with other known *Chlorella* species) was grown under non-axenic mixotrophic conditions using an acetic acid/pH auxostat feeding system in non-axenic conditions for 8 days. The experiment was conducted as a single batch with no harvesting during the 8 day period. The trial was performed in 2 foot by 2 foot flat panel air-sparged photobioreactors with a running volume of 14 liters (L). The volume was increased up to 15 L due to the addition of acetic acid solution. A control treatment was also run on a $CO_2$/pH auxostat system for a photoautotroph (phototrophy) culture. The reactors were inoculated at 0.1 g/L with 3 L of an exponentially growing monoalgal culture of *Chlorella sp. SNL 333.* The inoculum was obtained from an outdoor raceway pond running approximately at 0.5 g/L in a BG-11 culture medium. The cultures where then conditioned to the 2 foot by 2 foot flat panel photobioreactors until they attained a density of 1 to 2.5 g/L. The cultures were batch fed a nitrate-phosphate solution to maintain the nitrate level between 400 and 1500 ppm. The culture was maintained at roughly a constant pH of about 7.5, and a temperature of about 25 °C. The cultures were exposed to light from FT5-HO 8 bulb panels on each side of the reactor (one bulb = 259.6 $\mu$mol/m$^2$ s) in 24 h light periods (constant light), and aeration (0.7 liters air/ liter medium per min) in the treatment. To begin with, the cultures were started with 25% light intensity and $CO_2$ pH control until the cultures reached 0.5 g/L, and then with 50% light intensity until the culture reached 2 g/L. After the cultures reached 2 g/L, the treatment of controlling pH with acetic acid additions began. The $CO_2$ treatment comprised injection of $CO_2$ into the air stream at a ratio of 1 to 10 and was controlled by a solenoid and a pH controller set at 7.5.

The acetic acid treatment system comprised a solenoid, peristaltic pump, and pH controller set at 7.5 to control the acetic acid feed pumped into the reactor from the acetic acid feed tank. The acetic acid feed was diluted to between 10% and 50% concentration with water. A probe was tied to the feed tubing to ensure the pumping was steady and within a control range of pH fluctuation. Samples were taken every 24 s, with dry weight and ash free dry weight triplicates taken once a day. Contamination pictures were taken daily. Every 2-3 days a single non-destructive dry weight sample (centrifugation 200 ml) for freeze drying, fatty acid analysis was taken. The supernatant of the centrifugation was frozen for acetic acid analysis by gas chromatography. The acetic acid consumption was measured by monitoring the level in the acetic acid feeding tank.

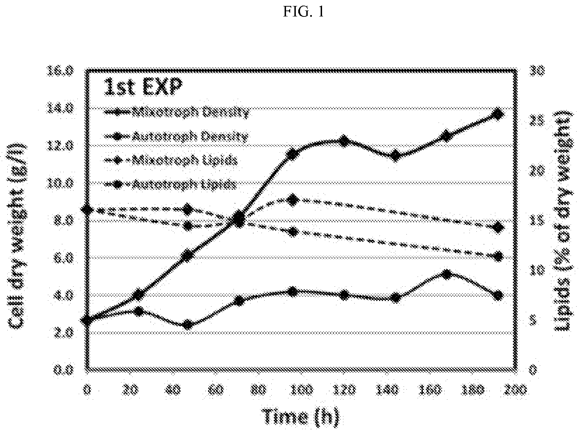

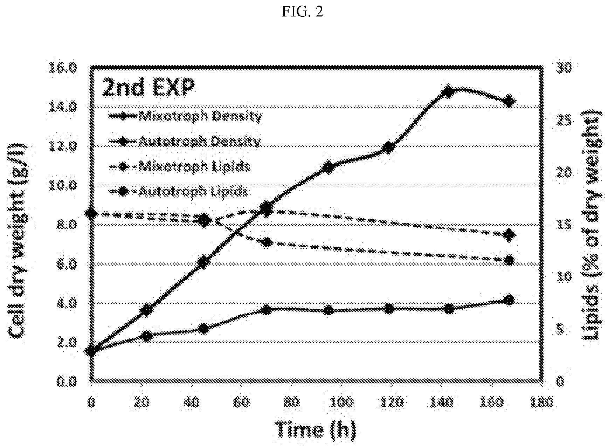

Referring to FIGS. 1-2 and Table 1, the results showed that the mixotrophic culture was more productive than the standard photoautotrophic (autotroph) culture with respect to cell dry weight (g/L). Table 1 lists the volumetric productivities and areal productivities for a mixotrophic and photoautotrophic culture of *Chlorella sp. SNL 333.* The results also showed that the mixotrophic culture had higher lipid content (% of dry weight) than the photoautotrophic culture. For the values listed in Table 1, the surface (illuminated) to volume ratio of the 2X2 feet reactor is 19 L/m$^2$. The areal productivity from the flat panel reactor experiment was calculated by multiplying volumetric productivities with the volume (19 L) that can be contained in 1 m$^2$. The extrapolated areal productivities grams per square meter per day (g/m$^2$ d) assumed 300 L/m$^2$ in mixotrophy. Additionally, mixotrophy, rather than phototrophy, was shown to be driving *Chlorella*'s mixotrophic growth. These results led to the conclusion that the surface (illuminated) to volume ratio is less critical for growth, and the limit for the amount of liters that can be placed in a square meter depends on the oxygen transfer of the system.

TABLE 1

| Productivity range | Growth Phase (2 d) | Whole batch (8 d) |
| --- | --- | --- |
| **Volumetric Productivities (g/L d)** | | |
| **Treatment** | | |
| Photoautotrophy | 0.5 | 0.4 |
| Mixotrophy | 2.6 | 1.6 |
| **Areal productivities (g/m2 d)** | | |
| Photoautotrophy | 9.5 | 7.6 |
| Mixotrophy | 49.4 | 30.4 |
| **Extrapolated Areal productivities (g/m2 d)** | | |
| Photoautotrophy | 9.5 | 7.6 |
| Mixotrophy | 780 | 480 |

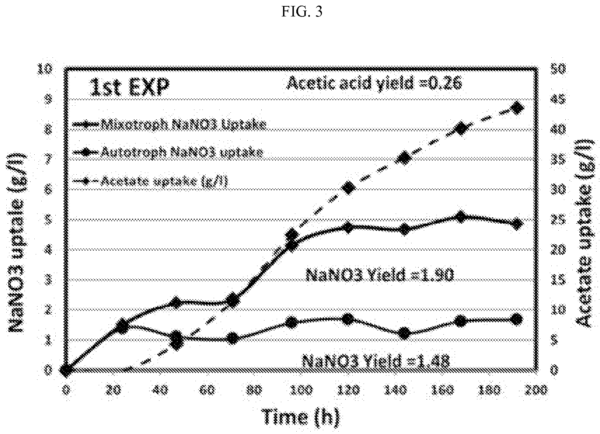

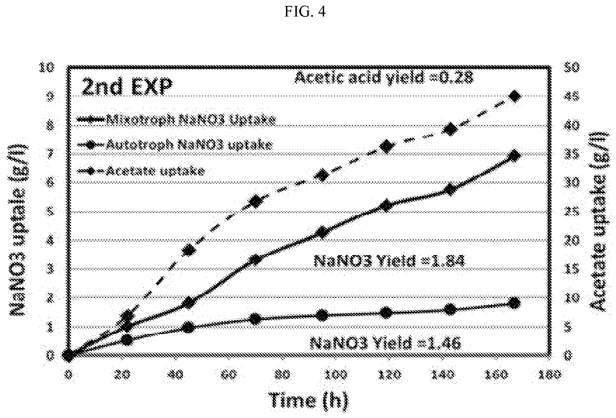

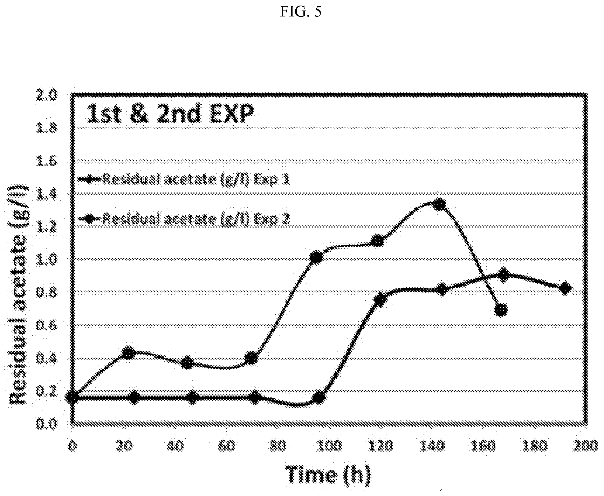

Referring to FIGS. 3-4, the results showed that the uptake of $NaNO_3$ was higher in the mixotrophic culture than the photoautotrophic (autotroph) culture. Referring to FIG. 5, the results showed that the residual acetic acid concentrations were relatively low (8 times less than table vinegar) in the mixotrophic culture using the acetic acid/pH auxostat system with a pH level set at 7.5, and would carry a low risk of environmental issues (spills, volatile organic carbon emissions), work hazards, or substrate waste. The residual acetic acid increased as nitrates and other nutrients were consumed.

Example 2

*Chlorella sp. SNL* 333 was grown under non-axenic mixotrophic conditions using an acetic acid/pH auxostat feeding system in non-axenic conditions for 10 days. The trial was performed with the same reactors and procedure as were used in Example 1, with the exception of the light exposure period (photoperiod), acetic acid feed system and the addition of nitrates only during growth (not phosphates or trace nutrients). The trials were also run as a single batch with no harvesting in the 10 day period, consistent with the procedure in Example 1. Trials were run with light exposure periods of 24hours in artificial light per day and with light exposure periods of 14 hours (10 dark per day), also in artificial light. The acetic acid was fed through a dripping system controlled by a needle valve in response to the pH level controller.

The dissolved oxygen levels were also measured continuously and updated in a data logger. Every four days microscopic photograph at 20X and 100X (oil immersion) were taken, as well as a measurement of cell counts, cell size, chlorophyll content, and the proportion of particles without chlorophyll.

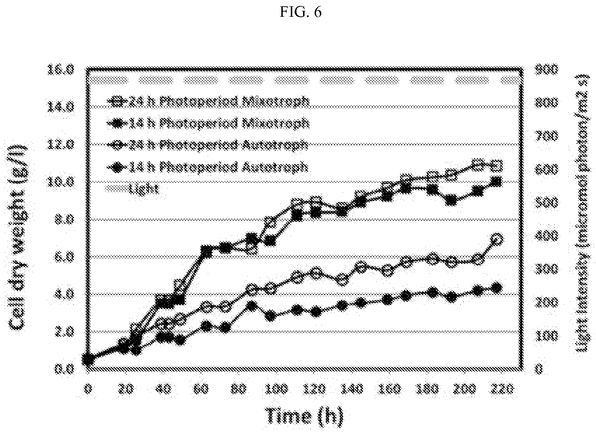

Referring to FIG. 6 and Table 2, the results showed the mixotrophic cultures out performed photoautotrophic (autotroph) cultures with respect to cell dry weight (g/L), and that the cell dry weight of the mixotrophic culture was less affected by the reduction in light exposure than the photoautotrophic culture. Table 2 lists the volumetric productivities and areal productivities for a mixotrophic and photoautotrophic culture of *Chlorella sp. SNL 333* grown at 24 h and 14 h photoperiod. For the values in Table 2, the surface (illuminated) to volume ratio of the 2x2 feet reactor is 19 L/m$^2$. The areal productivity from the flat panel experiment was calculated by multiplying volumetric productivities with the volume (19 L) that can be contained in 1 m$^2$. The extrapolated areal productivities (g/m$^2$ d) assumed 300 L/m$^2$ in mixotrophy. Additionally, mixotrophy, rather than phototrophy, was driving *Chlorella*'s mixotrophic growth based on the results.

TABLE 2

| Productivity ran ge | | Growth Phase (2 d) | Whole Batch (8 d) |
|---|---|---|---|
| Treatment | Photoperiod | Volumetric Productivities (g/L d) | |
| Photoautotrophy | 24 h | 1.06 | 0.70 |
| | 14 h | 0.66 | 0.47 |
| Mixotrophy | 24 h | 2.74 | 1.21 |
| | 14 h | 2.76 | 1.30 |
| | | Actual Areal productivities (g/m2 d) | |
| Photoautotrophy | 24 h | 20.1 | 13.3 |
| | 14 h | 12.5 | 8.9 |
| Mixotrophy | 24 h | 52.1 | 23.0 |
| | 14 h | 46.7 | 24.7 |
| | | Extrapolated Areal productivities (g/m2 d) | |
| Photoautotrophy | 24 h | 20.1 | 13.3 |
| | 14 h | 12.5 | 8.9 |
| Mixotrophy | 24 h | 822 | 363 |
| | 14 h | 828 | 390 |

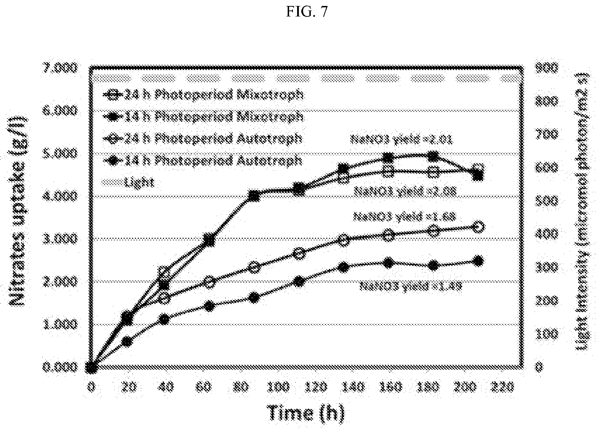

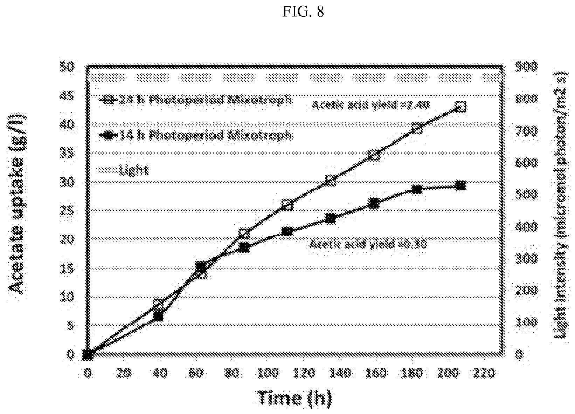

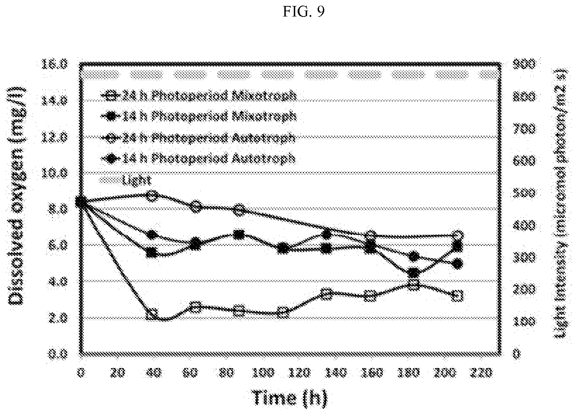

Referring to FIG. 7, the results showed that the NaNO$_3$ uptake was affected more by the reduction in the light exposure period for the photo autotrophic (autotroph) culture than for the mixotrophic culture. Referring to FIG. 8, the results showed that the acetic acid uptake in the mixotrophic cultures was lower for the 14 h light exposure than the 24 hour light exposure. Referring to FIG. 9, the results also showed that the dissolved oxygen level was under a possible critical point (20%) saturation, suggesting that the poor gas transfer of oxygen in the 2 foot by 2 foot flat panel photobioreactor was more critical to limiting the growth of the mixotrophic cultures than the light energy.

Referring to Table 3, the results showed that the bacteria levels in the mixotrophic culture were kept low despite the non-axenic conditions and introduction of an organic carbon source. Table 3 lists the incidence of bacterial populations in a *Chlorella sp. SNL* 333 culture operated under a mixotrophic or photoautotrophic regime. For the values listed in Table 3, algae cells were identified by chlorophyll self-florescence and bacterial cells were identified by backlight green die. The values of Table 3 also assumed a *Chlorella* weight of 27 x 10$^{-12}$ g/cell and bacterial cell weight of 0.2 x 10$^{-12}$ g/cell.

TABLE 3

| Bacterial population | | % of total cell counts*1 | % of total biomass*2 |
|---|---|---|---|
| Treatment | Photoperiod | | |
| Mixotrophy | 24 h | 4.9 | 0.04 |
| | 14 h | 3.9 | 0.03 |
| Photoautotrophy | 24 h | 1.6 | 0.01 |
| | 14 h | 1.6 | 0.01 |

Example 3

*Chlorella sp. SNL 333* was grown under non-axenic mixotrophic conditions using an acetic acid/pH auxostat feeding system in non-axenic conditions for 4 days. The trial was performed with the same equipment and procedure as previously described in Example 2, with the exception of the light exposure period (photoperiod) and the acetic acid feed. Trials one and two where run with 24 h light exposure and trials three and four were run with 0 h light exposure (heterotrophic). The acetic acid was fed at 200 g/L, and 1 g/L of initial sodium acetate for culture densities of 0.5 to 5-6 g/L; and acetic acid feed of 10 g/L at culture densities above 5-6 g/L. Acetic acid was fed into the auxostat in response to pH change. The reactor was fitted with an overflow pipe to allow the volume of culture exceeding the operating volume of the 2X2 (14 L) bioreactors to drain (harvest) into 4 L flasks for measurements and analysis.

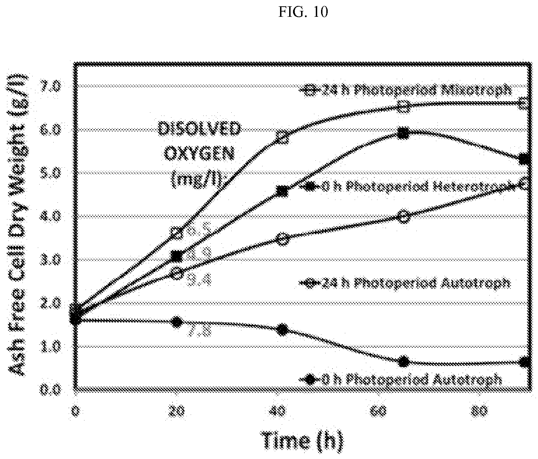

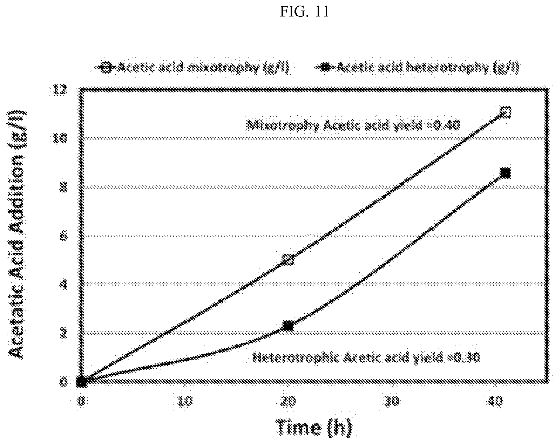

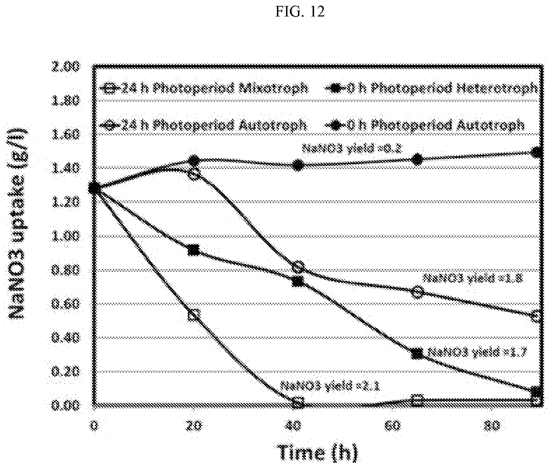

Referring to FIG. 10, the results showed that the *Chlorella* cultures fed with acetic acid grew better in the 24 h light exposure than the 0 h light exposure. Photosynthetic activity was also found to help improve the dissolved oxygen values from 4.9 mg/L for the near heterotrophic treatment (0 h light exposure with minimal ambient ingress of light) to 6.5 mg/L for the mixotrophic treatment (24 h light exposure). Referring to FIG. 11, the results showed the acetic acid consumption for the mixotrophic treatment (24 h light exposure) was higher than in the heterotrophic treatment (0 h light exposure). Referring to FIG. 12, the results showed that the residual $NaNO_3$ concentration was lower for the mixotrophic culture (24 h light exposure) than for the near heterotrophic culture (0 h light exposure) or the photoautotrophic (autotroph) cultures.

Example 4

*Chlorella sp. SNL 333* was grown under non-axenic mixotrophic conditions using an acetic acid/pH auxostat feeding system for 10 days. The trial was performed with two raceway pond photobioreactors made of PVC, with a cultivable area of 5.6 $m^2$ and a 10 cm light path (i.e. culture depth). Both photobioreactors contained mixotrophic cultures, aerated with two 50 cm porous diffusers at 10 liters per minute (LPM), and were located outdoors. The first photobioreactor (Reactor 1) was mixed hydraulically (pump). The second photobioreactor (Reactor 2) was mixed with a paddle wheel. *Chlorella sp. SNL 333* was inoculated at a density of 0.3 g/L in the Reactors 1 and 2. The *Chlorella* was adapted to the outdoor conditions under $CO_2$/pH control until it attained a density of 0.3-0.4 g/L for the experimental trials. The acetic acid additions occurred using the drip system previously described in Example 2.

The cultures were harvested as needed when the culture density reached 1.5 g/L. The initial medium was a BG-11 medium with sodium acetate (1 g/L) supplemented in the initial medium. Natural sunlight was applied to the culture, with the average photoperiod in May 2012 for Gilbert, AZ being about 14.5 h. The temperature was controlled by cooling coils at 28 °C. The pH controller of the pH auxostat system, as previously described, was set at 7.5. Temperature, pH and dissolved oxygen were measured continuously. Acetic acid consumption was monitored by the acetic acid feed tank level daily. Dry weights were taken three times (n=3) daily and nitrate levels were taken daily. Spin down to measure residual acetate (200 ml) and biomass was performed every two days.

Contamination observation (400X, 1000X oil immersion-phase contrast micrograph and cell cytometry with bacterial dying) was performed every 2 days, including a measurement of bacterial contamination by flow cytometry. For bacterial contamination measurement, to each 1 ml sample, 1 $\mu$l BacLight™ Green bacterial stain (Invitrogen, Eugene, OR, USA) as added, and samples were incubated at room temperature in the dark for 30 to 60 minutes. After incubation, samples were analyzed on a BD FACSAria™ (BD Biosciences, San Jose, CA, USA) and populations of bacteria and algae were gated based on BacLight™ fluorescence and chlorophyll autofluoresence.

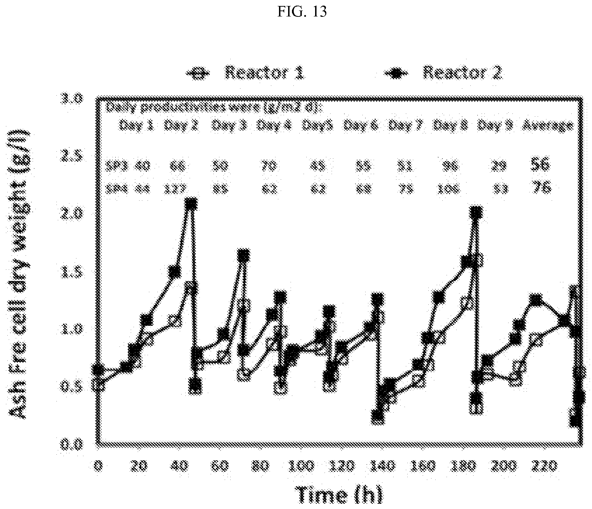

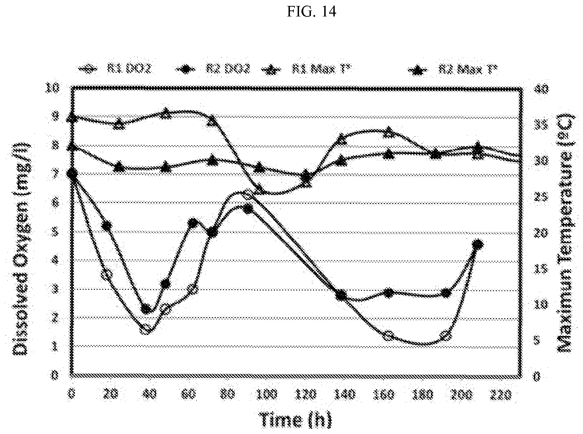

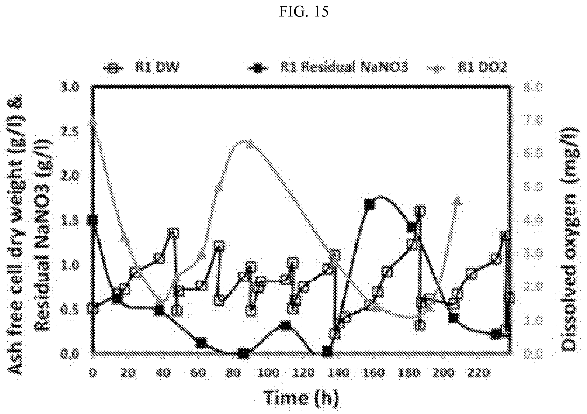

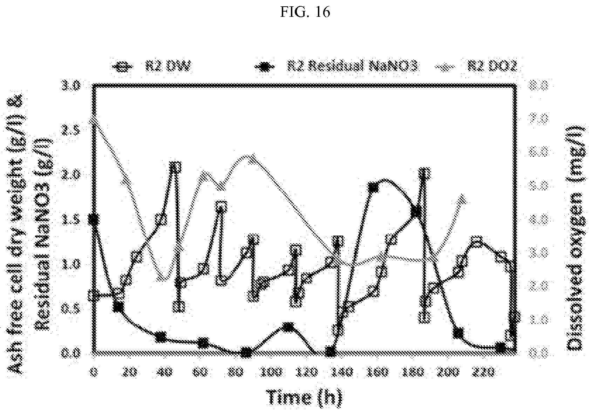

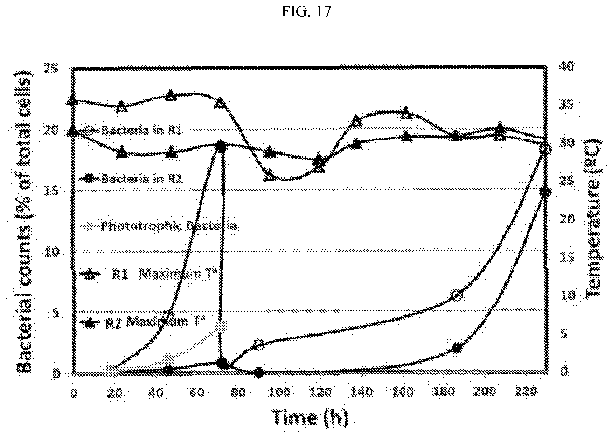

Referring to FIG. 13, results showed a maximum daily productivity of 97 g/$m^2$ d (0.97 g/L d) for Reactor 1 (i.e., SP3) and 127 g/$m^2$ d (1.27 g/L d) for Reactor 2 (i.e., SP4). The average daily productivity (over 9 days) was 56 g/$m^2$ d (0.56 g/L d) for Reactor 1 and 76 g/$m^2$ d (0.76 g/L d) for Reactor 2. The productivity of the mixotrophic cultures in the outdoor reactors was roughly six times the productivity of the photoautotrophic cultures previously obtained in the outdoor reactors. Referring to FIGS. 14-16, the results showed Reactor 1, which had the lower productivity than Reactor 2 (R2), also had the lower dissolved oxygen level, which corresponds to the previous finding that relates low dissolved oxygen levels with growth limitation. Referring to FIG. 17, the results showed that bacteria levels were below 5% of total cell counts (<0.05% of total biomass) in the outdoor, non-axenic mixotrophic conditions when the temperature was maintained below 30 °C.

Example 5

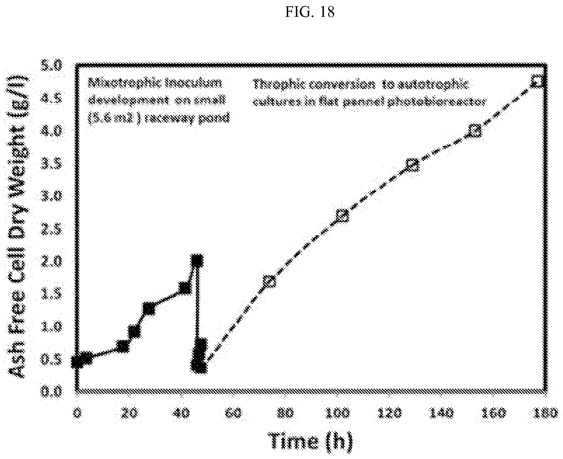

*Chlorella sp. SNL 333* (was grown mixotrophically in non-axenic conditions in outdoor, open raceway pond photobioreactors using an acetic acid/pH auxostat system for 10 days before being transferred to flat panel photobioreactors for photoautotrophic (phototrophic) growth. The outdoor reactors were operated as described above in Example 4. The flat panel photobioreactors were inoculated with a culture of *Chlorella* from the outdoor reactors at a density of 0.5 g/L, and operated at an average temperature of 25 °C, pH of 7.5 controlled by $CO_2$, aeration at 10 LPM, $CO_2$ pulses at 2 LPM, light exposure (photoperiod) of 14 h from FTS-HO 8 bulb panels (one bulb = 259.6 $\mu$mol/m$^2$ s) on each side of the photobioreactor. Data collected once a day included: optical density at 750 and 680 nm and pH. Dry weight measurements, nitrate measurements, and chlorophyll analysis was performed at the beginning, middle and end of the trial. Referring to FIG. 18, the results showed that the mixotrophically grown inoculum from the outdoor reactors matched typical photoautotrophic (autotrophic) growth rates after the transfer to the flat panel photobioreactors for photoautotrophic growth, and that the trophic conversion was instantaneous.

Example 6

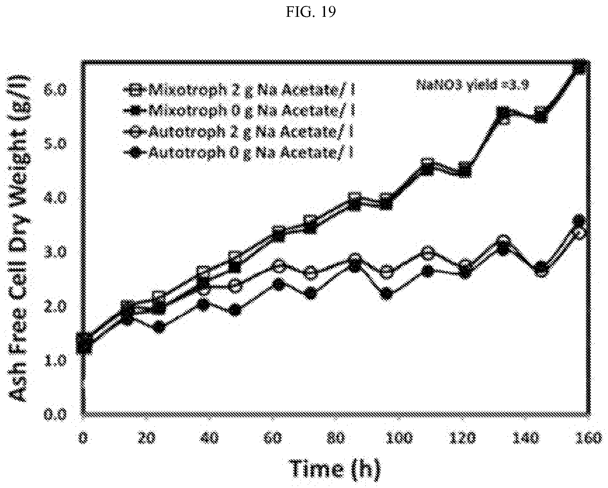

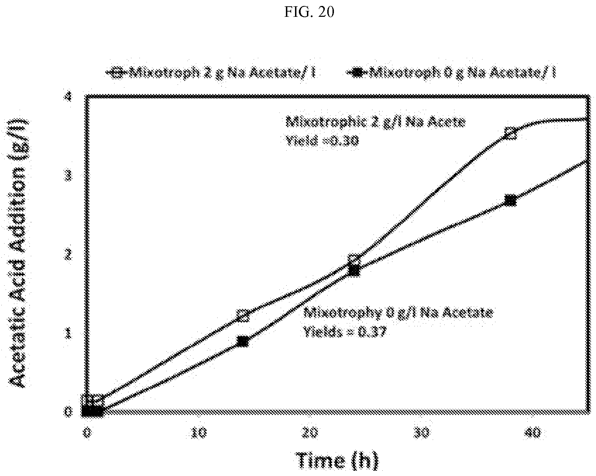

*Chlorella sp. SNL 333* was grown mixotrophically in non-axenic conditions using an acetic acid/pH auxostat system and the initial addition of sodium acetate to the culture medium. The equipment and procedure as previously described in Example 2 was used to culture the *Chlorella* mixotrophically with a 14 h light exposure (photoperiod). The first and fourth trials received 2 g/L of sodium acetate initially, and the second and third trials received no sodium acetate initially. Acetic acid was fed at 200 g/L. Dry weight, ash free dry weight, dissolved oxygen, acetic acid, bacterial contamination, and nitrates were monitored as described in previous experiments. Referring to FIGS. 19-20, the results showed that the residual acetic acid mirrors the buffering requirement of the culture medium, which are determined by the consumption of the mineral salts (nutrients), the consumption of $CO_2$ by photosynthetic processes, as well as the excretion of organic acids by the cell. Therefore the system is not purely an auxostat (constant concentration), but varies the concentration $\pm 0.5$ g acetic acid/L within the batch. The results also showed that the initial sodium acetate ensured the presence of acetate at all times and a successful activation of the pH control regardless of the photosynthetic process.

Example 7

*Chlorella sp. SNL 333* was cultivated in open pond reactors, in non-axenic conditions using an acetic acid/pH auxostat system. The equipment R1 and procedure as previously described in Example 4 was used with the exception that sodium acetate was not initially added to the culture medium. The results showed a failure in the activation of the acetic acid/pH auxostat system and productivities equivalent to those obtained in photoautotrophic system.

Example 8

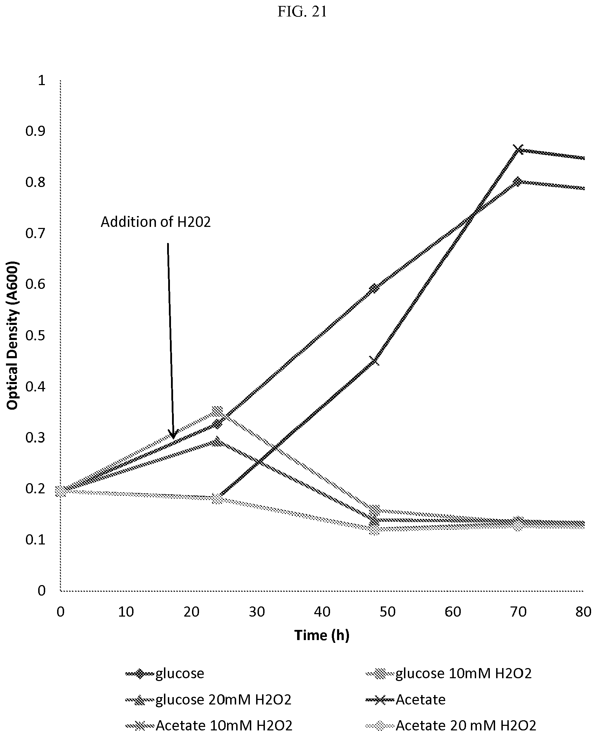

Hydrogen peroxide was applied to a culture of *E. coli* to determine the inhibitive effects on the bacteria. 200 ml of bacteria inoculum from a bacteria contaminated photobioreactor was added to 200 ml of BG-11 culture medium. The solution was split in half, with the first solution receiving 6.84 g of glucose and the second solution receiving 5 g of sodium acetate. The pH and optical density of both solutions was taken. The two solutions were each split into three groups of three separate 100ml volumes, for a control, 10 mM$H_2O_2$, and 20 mM$H_2O_2$ treatments. Each volume had 2.092 g of MOPS buffer (Sigma Chemical St. Louis, MO), and then placed in an incubator set at 27 °C, 96 rpm, and 100 $\mu$mol/m$^2$ s of LED light. The volumes were incubated overnight, then brought to a pH of 7.5 using 10 M NaOH and optical density was measured. The treatments of 10 mM $H_2O_2$ and 20 mM $H_2O_2$ were then added and incubated for 24 h. Optical density and pH were then measured, and methionine was added to one volume of each treatment. Optical density and pH were measured again on the third day and the sixth day before microscope analysis on the sixth day. The results showed that the addition of $H_2O_2$ had little effect on the pH of the culture. Referring to FIG. 21, the results also showed that the addition of $H_2O_2$ negatively affected the growth of bacteria cultures in both glucose and acetate mediums, with the 20 mM $H_2O_2$ treatment having a greater effect than the 10 mM $H_2O_2$ treatment.

Example 9

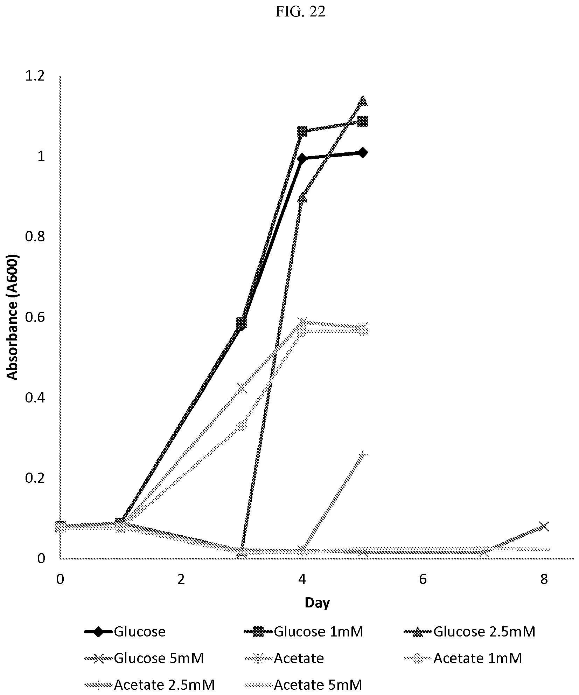

Contaminating bacteria were isolated from microalgae cultures and identified as *Escherichia coli.* Different concentrations of hydrogen peroxide were applied to cultures of *E. coli* to determine the inhibitive effects on the bacteria using the same equipment and procedures as were used in Example 8. The bacteria cultures in glucose and sodium acetate were treated with 0 mM $H_2O_2$ (control), 1 mM $H_2O_2$, 2.5 mM $H_2O_2$, and 5 mM $H_2O_2$. Referring to FIG. 22, the results showed that as the concentration of $H_2O_2$ increased the inhibitory effect on the growth of the bacteria increased. The results also showed that the bacteria culture growth would eventually recover from the one time treatment, indicating that continued treatment would be necessary to control the bacteria population. Due to the recovery of the bacteria, periodic dosing would aid in controlling the bacteria population. For cultures containing glucose: a 2.5 mM $H_2O_2$ treatment should be dosed every 2 days, or every 6 days if a 5 mM $H_2O_2$ treatment is used. For cultures containing sodium acetate, a 2.5 mM $H_2O_2$ treatment should be dosed every 3 days, suggesting the higher sensitivity of acetate feed bacteria to oxidative stress than the glucose feed algae.

Example 10

*Chlorella sp. SNL333* was grown in a mixotrophic system in the pond raceway systems described above in Example 4. The area of the raceway photobioreactor was 5.6 m$^2$ with an operating volume of 568 L at a depth of 15 cm. One unit was mixed with a pump (Reactor 1), and the other via a paddle wheel (Reactor 2). Air was delivered to the paddle wheel system via an air stone. Air was delivered to the pumped reactor via a venturi injection system. Eductors were used in the pumped reactor to increase water velocity and enhance oxygen transfer of the liquid medium. The growth rate is proportional to the amount of oxygen available. The oxygen required for the estimated productivity of 20 - 200 g /m2 d would be approximately 34 - 100 g/ m$^2$ d. Reactor1 and Reactor 2 were inoculated with *Chlorella* at 0.48 g/L. Sodium acetate was added to the culture medium of the starting culture on day 0 at a concentration of 0.3 g/L. The nutrient feed to the system consisted of 20% acetic acid and a BG-11 nutrient solution with modified nitrate levels (350 mg/ L). The nitrate level in the modified BG-11 recipe consisted of 400 mg/L, and was determined via the nitrate consumption rates from previous trials.

During the trial the residual nitrate in both systems reached 0 a few days through the trial. Nitrate levels were adjusted towards the end of the trial to maintain residual nitrate in the system (400 mg/L). Harvests occurred daily throughout the trials. Harvest consisted of 50% of the culture volume daily, with an 80% harvest occurring on day 7. The total trial lasted 12 days for Reactor 1 and 10 days for Reactor 2.

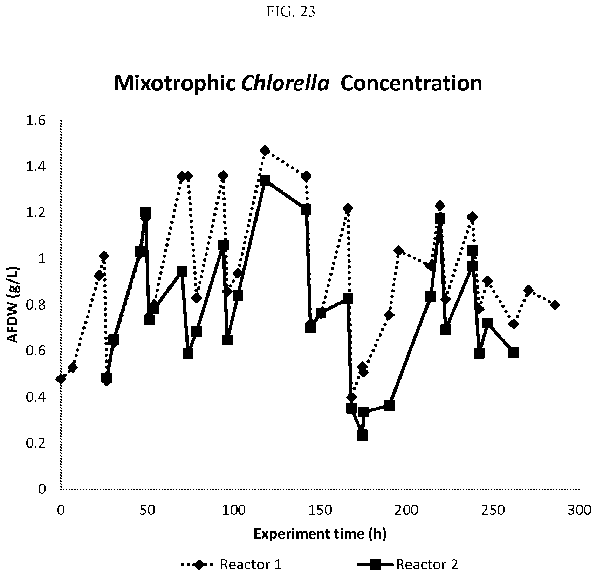

Referring to FIG. 23, the results showed the highest concentration of biomass reached in Reactor 1 was 1.47 g/L (120 h) and in Reactor 2 was 1.34 g/L (120 h). Biomass concentration is displayed as ash-free dry weight (AFDW) in g/L. The system was harvested when concentrations reached 1.0 g/L or greater. Harvests consisted of 50% of the total culture volume per day, with an 80% harvest occurring on day 8 for both systems.

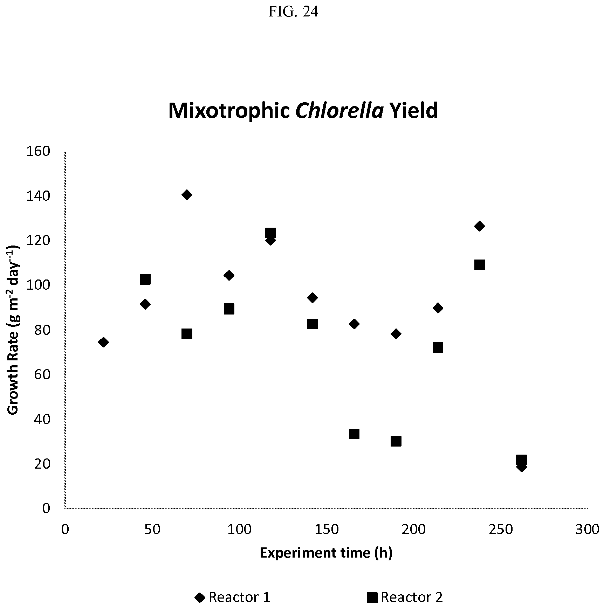

Referring to FIG. 24, the results showed the yield in g/ m$^2$ d for the raceway mixotrophic systems Reactor 1 outperformed Reactor 2 on a total culture length basis and total average daily yield. Reactor 1 culture ran for a total of 12 days, and Reactor 2 culture ran for a total of 10 days. The average daily yield for Reactor 1 over the 12 day period was 87 g/m$^2$ d (includes last two days when culture viability decreased due to bacterial contamination). The average daily yield in Reactor 1 for the first 10 days was 101 g/m$^2$ d. The average daily yield for Reactor 1 over the 10 day period was 76 g/m$^2$ d (includes last two days when culture viability decreased due to bacterial contamination). The average daily yield in Reactor 2 for the first 8 days was 74 g/m$^2$ d.

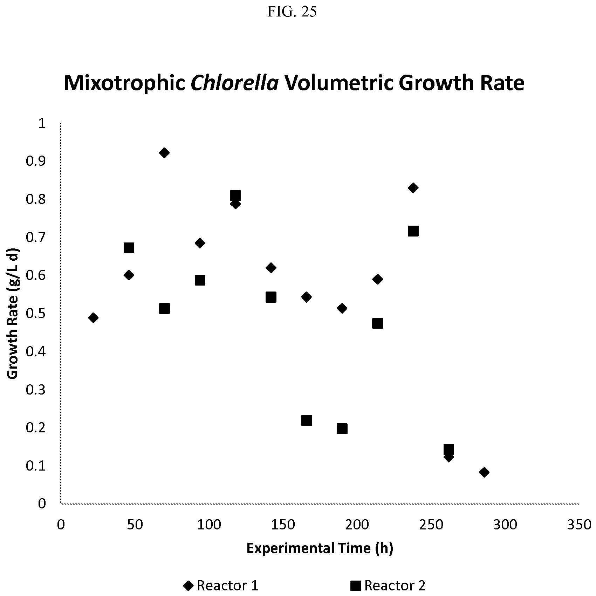

Referring to FIG. 25, the volumetric growth rate results of Reactor 1 and Reactor 2 are shown. The average volumetric productivity in Reactor 1 was 1 g/L d for the 12 day trial and 0.66 g/L d for the first 10 days of the trial. The maximum volumetric productivity achieved in Reactor 1 during the trial was 0.92 g/L d. The average volumetric productivity in Reactor 2 was 0.49 g/L d for the 10 day trial and 0.50 g/L d for the first 8 days of the trial. The maximum volumetric productivity achieved in Reactor 2 during the trial was 0.81 g/L d.

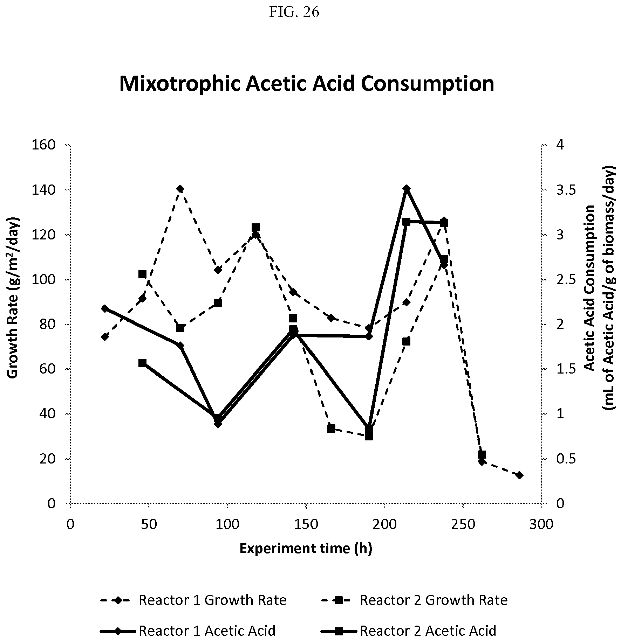

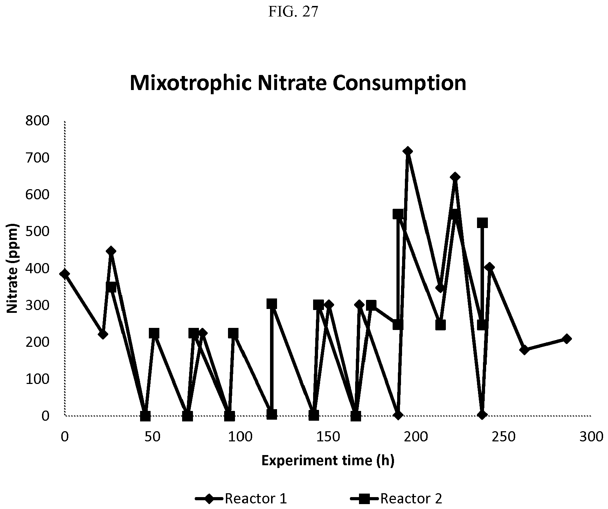

FIG. 26 shows the results yield and the acetic acid consumption during the trials. Reactor 1 consumed on average 2.31 L/d of acetic acid over the first seven days. Reactor 2 consumed on average 1.83 L/d of acetic acid over the first six days of the trials. FIG. 27 shows the nitrate consumption under mixotrophic conditions. Reactor 1 consumed a maximum of 447 mg/L d of nitrate. Reactor 2 consumed a maximum of 350 mg/L d.

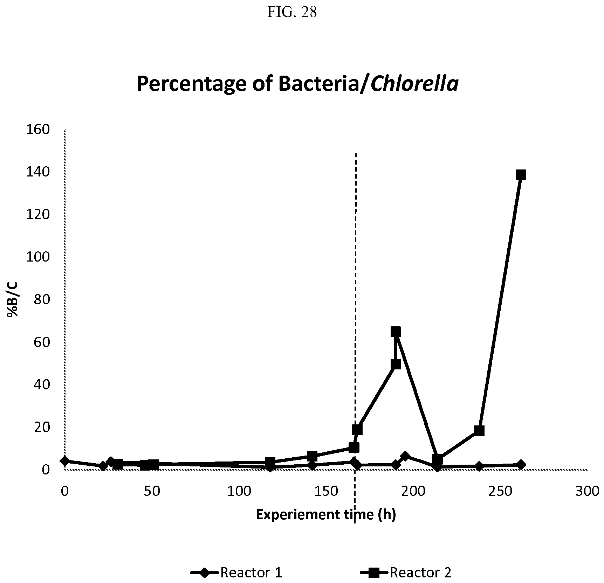

Referring to FIG. 28, the percentage of bacteria to *Chlorella* in the mixotrophic cultures was quantified using the procedure discussed in the previous examples. Treatments to control the bacterial population occurred at the time indicated by the vertical dashed line. Reactor 1 received an ozone treatment, and Reactor 2 received an antibiotic treatment. At 190 hours the make-up water for refilling the system was sterilized by chlorination prior to being added to the culture. In Reactor 1 the percentage of bacteria to *Chlorella* in the culture had reached 10% at 240 h. In Reactor 2 the percentage of bacteria to *Chlorella* in the culture had reached 18% at 240 h. At 168 h the bacteria levels in both Reactor 1 and Reactor 2 began to rise and both systems were treated. Reactor 1 was treated with ozone applied at average levels ranging from 0.10 - 0.20 mg/L for 17 h. The ozone was injected into an air sparger and mixed with air at a PSI of 5. Reactor 2 was treated with an antibiotic mix as listed in Table 4. The percentage of bacteria to *Chlorella* was reduced from 6.5% to 1.4% in Reactor 1 using the ozone treatment. The percent of bacteria in Reactor 2 after the antibiotic treatment was reduced from 65% to 19%. The average yield in Reactor 1 for the three days after the ozone treatment was 105 g/m$^2$ d. The average yield in Reactor 2 for the three days after the antibiotic treatment was 71 g/m$^2$ d. The antibiotic treatment reduced bacteria in the system but also reduced productivity. The ozone treatment reduced bacteria in the system and maintained a yield of over 100 g/m$^2$ d.

TABLE 4

| Antibiotic | Target Concentration | Actual Concentration | Re-suspended in: |
|---|---|---|---|
| Penicillin | 250 mg/L | 150 mg/L | Water |
| Tetracycline | 62.5 mg/L | 16.1 mg/L | Ethanol |

(continued)

| Antibiotic | Target Concentration | Actual Concentration | Re-suspended in: |
|---|---|---|---|
| Chloramphenicol | 7.5 mg/L | 7.5 mg/L | Ethanol |
| Aureomycin | 6.25 mg/L | 6.25 mg/L | Methanol |

Example 11

The oxygen demand for a mixotrophic microalgae culture was calculated for the outdoor, open raceway pond reactors previously described in Example 4. The calculated growth rate from Example 10 was measured as 0.6 g/L d on an average over 9 days of continuous operation. Approximately 33% of acetic acid by mass is converted to biomass which equates to 3.33 g acetic acid /g of biomass produced. The theoretical stoichiometric consumption of oxygen to consume acetic acid is as follows:

$$CH_3COOH + 2O_2 \longrightarrow 2CO_2 + 2H_2O$$

1 mole acetic acid requires 1.5 moles oxygen or 60 g acetic acid requires 64 g $O_2$ or 1.07 g $O_2$/g acetic acid.

Alternate intermediates other than $CO_2$ are possible for conversion into biomass and not explicitly noted here. Carbon sources other than acetic acid, such as but not limited to glucose, are also possible in a mixotrophic culture. The calculated oxygen consumption rate is as follows: (0.6 g biomass/L d x 3.33 g acetic acid/g biomass x 1.07 g $O_2$/g acetic acid) = 2.14 g $O_2$/L d, which calculates to a consumption rate of 1.48 ppm $O_2$/min.

Equilibrium oxygen concentration at 25 °C is 8.5 ppm, hence the calculated $k_La$ from the experiment described in Example 9 is 0.17 min$^{-1}$ or 2.90 x 10$^{-3}$ s$^{-1}$.

Example 12

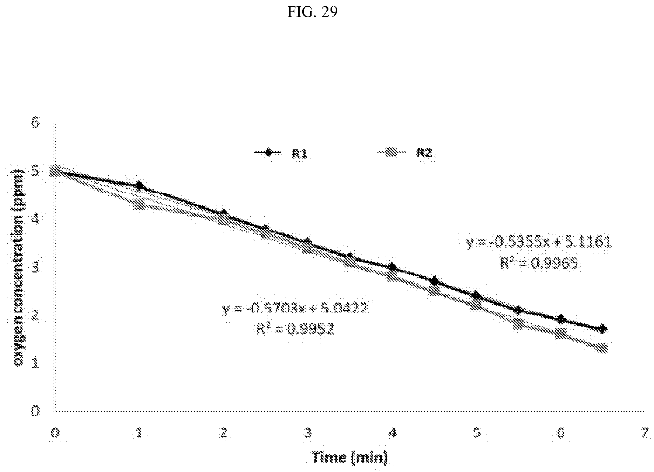

An additional experiment was conducted on a small sample removed from the open, outdoor reactors as described in Example 4. The culture on the open pond was at equilibrium at a dissolved oxygen level below saturation, suggesting that the oxygen consumed was equal to the oxygen transferred. Two different tests were performed on samples from open, outdoor reactors. 1L samples were brought in the lab and covered with cloth to block the light. The rate of oxygen usage was measured with a dissolved oxygen probe (DOH-SD1™ polarographic dissolved oxygen meter, OMEGA Engineering Ltd Manchester UK), and the data is shown in the FIG. 29. The culture from the tanks was used to fill a 250 ml Erlenmeyer, and to reduce the oxygen transfer. The drop of DO level was plotted against time in order to calculate the oxygen demand (at DO level above critical level).

From FIG. 29, the rate of consumption of $O_2$ was shown to be the slope of the curve 0.54 ppm/min for Reactor 1 and 0.57 ppm/min for the Reactor 2 sample. The initial concentration was 5 ppm $O_2$ and so the concentration gradient for the test would be 8.5 to 3.5 ppm. The $k_La$ in this case for Reactor 1 would be between 3.5 s$^{-1}$ and 2.75 10$^3$ s$^{-1}$.

Example 13

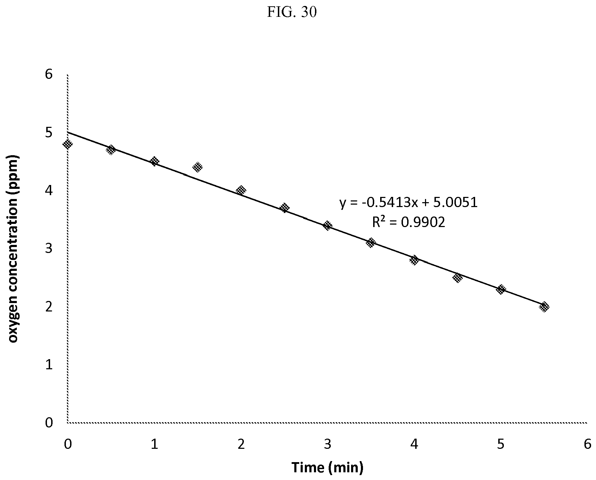

A test as described in Example 12 was performed on a different day from Reactor 1 and results are shown in FIG. 30. The graph in FIG. 30 plots the oxygen consumption rate as measured in Reactor 1 during the period of growth with an average volumetric growth rate of 0.6 g/L d or 100 g/m$^2$ d for the 15 cm deep reactor. The oxygen consumption rate of the sample was 0.5413 ppm/min. The initial concentration was 4.8 ppm, and so the concentration gradient for the test would be 8.5-4.8~ 3.7 ppm. The $k_La$ in this case for Reactor 1 would be 0.107 min$^{-1}$ or 2.44 x 10$^{-3}$ s$^{-1}$. The average $k_La$ for the experiment was 2.7 x 10$^{-3}$. The results for the $k_La$ calculations are summarized in Table 5.

TABLE 5

| Date | Theoretical calculation | Day 1 | | Day 2 |
|---|---|---|---|---|
| Reactor Sampled | | Reactor 1 | Reactor 2 | Reactor 1 |
| OUR (ppm/min) | 1.48 | 0.54 | 0.57 | 0.54 |
| C* ppm | 8.5 | 8.25 | 8.5 | 8.5 |
| C ppm | 0 | 5 | 5 | 4.8 |

(continued)

| Date | Theoretical calculation | Day 1 | | Day 2 |
|---|---|---|---|---|
| *Reactor Sampled* | | *Reactor 1* | *Reactor 2* | *Reactor 1* |
| $k_La$ (s$^{-1}$) | 2.90E-03 | 2.75E-03 | 2.72E-03 | 2.44E-03 |
| C* is concentration of saturation, C is oxygen concentration | | | | |

Example 14

In a prophetic example, the areal growth rate of microalgae or cyanobacteria biomass for a mixotrophic reaction can range from 100 to 10,000 g/m$^2$ d, with a more expected range from 100 to 4,000 g/m$^2$ d. The volumetric growth rate may range from 0.6 g/L d to 600 g/L d, with a more expected range from 0.6 to 6 g/L d. The prophetic microorganism growth reactors may be closed or open and operate outside or inside. The inside reactors may be operated with external light around the reactor or a light pipe or other means of inserting light within the volume of a microalgae growth reactor.

The reaction of acetic acid with oxygen and microalgae is volumetric in nature and limited by interfacial mass transfer. As described in Example 2, the indoor volumetric growth rate was 2.7 g/L d. This experiment was limited by oxygen mass transfer into the liquid as seen by the low value of dissolved oxygen. For Example 2, oxygen was fed in the form of air through a simple sparger made from a tube with spatially distributed holes of approximately 1/32 inch (.079375 cm) diameter spaced every 0.75 (1.905 cm) inch and staggered between two rows (approximately 32 holes total).

With smaller air injection points, an increased number of injection points, and/or an increase in the breakage of larger bubbles, more oxygen may dissolve into the microalgae culture solution. With more dissolved oxygen in the microorganism culture solution, a higher volumetric mixotrophic growth rate of microalgae or cyanobacteria may be achieved. With an air injection diameter less than 0.1 mm and more preferably between $1\times10^{-7}$ m and $1\times10^{-4}$ m, the corresponding volumetric growth rate will exceed 2.7 g/L d and range from 0.6 g/L d to 600 g/L d.

Further, increased oxygen solubility or uptake into the liquid reaction media may also be achieved with an increase in pressure. One means for increasing pressure is the use of liquid column height. In one prophetic example, a reactor ranging from 0.2 m to 40 m tall may be used, with a preferred range from 0.2 m to 10 m tall. This reactor may be tubular in nature and have air, enhanced oxygen air from a pressure swing absorption (PSA) generator or other unit operation, such as but not limited to membrane separation, which delivers a gas with an oxygen composition ranging from 25% to about 98%, or high purity oxygen fed at or near the bottom of the reaction vessel. The levels of oxygen may be controlled to enhance growth of microalgae or cyanobacteria and to control contamination organisms. An inventive microalgae or cyanobacteria farm may be comprised of banks of 0.2 m to 40 m tall tubular reactors with air fed at the bottom and optionally including a center or spatially distributed light pipe. The light pipe may preferentially be made from wavelength specific LED's comprised of any wavelength that may enhance growth and/or photopigment product formation, and may alternatively be disposed around the exterior of the tube. The tube diameter may range from 0.1 m to 10 m. The inventive algae farm may be housed outdoors or inside an enclosure to avoid challenges from inclement conditions, including inconsistent solar radiation, UV degradation, bugs, animals, contamination, windstorms, rainstorms, haboobs, hurricanes and the like.

Alternately, a water or culture medium column may be adjacent to a reactor (e.g., tubular, flat panel, or substantially planar reactor), whereby the higher dissolved oxygen may be added to the media in the tall column either before addition to the inventive reactor or cycled to and from the reactor to increase the amount of dissolved oxygen to aid the reaction.

Alternately, a substantially planar and substantially tubular reactor are used together, where a first stage tubular reactor is used for high growth and a second substantially planar reactor used for lipid or pigment production for a microalgae strain that may comprise of species from the genera such as: *Agmenellum, Amphora, Anabaena, Anacystis, Apistonema, Pleurochrysis, Arthrospira (Spirulina), Botryococcus, Brachiomonas, Chlamydomonas, Chlorella, Chloroccum, Cruciplacolithus, Cylindrotheca, Coenochloris, Cyanophora, Cyclotella, Dunaliella, Emiliania, Euglena, Extubocellulus, Fragilaria, Galdieria, Goniotrichium, Haematococcus, Halochlorella, Isochyrsis, Leptocylindrus, Micractinium, Melosira, Monodus, Nostoc, Nannochloris, Nannochloropsis, Navicula, Neospongiococcum, Nitzschia., Odontella, Ochromonas, Ochrosphaera, Pavlova, Picochlorum, Phaeodactylum, Pleurochyrsis, Porphyridium, Poteriochromonas, Prymnesium, Rhodomonas, Scenedesmus, Skeletonema, Spumella, Stauroneis, Stichococcus, Auxenochlorella, Cheatoceros, Neochloris, Ocromonas, Porphiridium, Synechococcus, Synechocystis, Tetraselmis Thraustochytrids,* and *Thalassiosira.* In one example, a high growth first step tubular reactor may be used for a culture of *Haematococcus* to produce astaxanthin. The second substantially planar reactor may be used in a final step to increase the production of astaxanthin as additional light may be used to induce the strong red color. A substantially planar reactor may take the form of a raceway pond of any width, length or depth up to about 1 m.

As described by Yue et al, Chemical Engineering Science 62 (2007) 2096-2108, a gas-liquid $k_La$ of 21 s$^{-1}$ for $CO_2$ in water has been described in the literature using process intensification techniques. A factor of 10,000 times higher mass transfer of gas into a liquid is possible than the calculated value from the disclosed mixotrophic examples. As the rate of oxygen mass transfer increases, other reaction phenomenon or inherent microalgae conversion rates may become limiting, and as such 6,000 g/L d (or 10,000 times higher rates than described here) may not be possible. It is theorized that up to 600 g/L d may be possible to achieve in a continuous mixotrophic culture with the use of improved gas-liquid mass transfer into a microalgae growth culture.

From the experiment described in Example 10 an areal productivity of 101 g/m2 d was measured with a corresponding volumetric growth rate of 0.66 g/L d. With improved mass transfer of 100 times, which is still well below the theoretical possibility of 10,000 times, and with an increase in reactor depth from 15 cm to 150 cm (or a 10 times increase in reactor volume for the same displaced surface area exposed to the sun) while still maintaining a mixing regime to allow at least some access to light for the entire culture, then the resultant areal growth would be three orders of magnitude higher than the current reported experiments or up to 10,000 g/m$^2$ d. With a lower culture depth or less optimized gas-liquid mass transfer, then the range of areal productivity from a mixotrophic culture may range from 100 g/m$^2$ d to 1,000 g/m2 d.

Example 15

An experiment was conducted in a raceway pond bioreactor with a total volume of 6600 L, a depth of 15 cm, and a length of roughly 80 feet (23.4 m). Two of the bioreactors were housed inside of a greenhouse to limit the ingress of dirt and contaminants, as well as mitigate the effect of weather induced events. The reactor was inoculated with *Chlorella sp. SNL 333* at 0.2 g/L initial density. The pH was controlled with acetic acid (20 % solution in water) following the procedures described above for acetic acid dosing. The reactors were mixed hydraulically and aerated with porous hoses. The system was operated semi-continuously with 50% of the cultures being harvested daily to maintain the cell density between 0.5 and 1.5 g/l dry weight. Cell dry weight of the cultures was measured before and after each harvest. The reactors were operated in duplicates for 6 consecutive days before completely harvesting the cultures. Bacterial counts were maintained below 5 % of total cell counts during the cultivation period. The system productivities exceeded 100 g/m$^2$ d showing comparable results to the smaller volume bioreactors described in Example 10.

Example 16

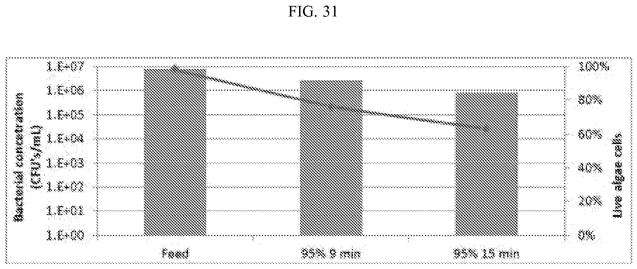

An experiment was conducted to determine the effect on mixotrophically grown microalgae (*Chlorella sp. SNL 333)* in culture with contamination bacteria. A UID 400 sonicator from Hielscher Ultrasonics GmbH (Teltow, Germany) was used to apply sonic energy to a 600 ml culture of mixotrophically cultured microalgae and bacteria. The culture was sonicated at 95% intensity using a 30 kHz frequency horn for 1.5 minutes increments and cooled to maintain the culture temperature in a range of 25-35 °C. The culture was re-inoculated into a small test system after 15 minutes of sonication treatment, and the growth rate of the treated and untreated microalgae were found to be within 10%. Referring to FIG. 31, the columns represent the bacterial concentration and the line represents the percent of live algae cells (i.e., microalgae cells). The results showed a log reduction in the bacterial concentration was achieved after 15 minutes of sonication treatment without a significant loss of live microalgae cells.

Example 17

A mixotrophic bioreactor system comprising a raceway pond providing the portion of the bioreactor system receiving at least some light, and a protein skimmer (i.e., column apparatus using gas injection to conduct foam fractionation) providing a portion of the bioreactor system which received no light, was used to cultivate *Chlorella* in mixotrophic culture conditions in an aqueous culture medium. The raceway pond held a volume of 500 L at an operational culture depth of 30 cm and was in fluid communication with a high flow venturi-pumped RK2RK75 protein skimmer with an adjustable operating volume of 408-466 L, resulting in a total bioreactor system volume of 908-966 L. The microorganism culture was circulated by pumps in the raceway pond, and exited the raceway pond through an outlet in fluid communication with the protein skimmer. Air injection for manipulation of the dissolved oxygen concentration of the culture was performed by the protein skimmer venturi pump. Acetic acid was dosed to the microorganism culture at a 20% concentration by an omega metering pump into the discharge line of the protein skimmer, which returned the microorganism culture to the raceway pond and completed the culture circulation path between the protein skimmer and raceway pond portions of the bioreactor system.

The pH level was sensed by Hannah pH probes in the raceway pond at the outlet to the protein skimmer and at the inlet from the protein skimmer. Eutech dissolved oxygen probes were mounted in the protein skimmer inlet and discharge pipes to detect the dissolved oxygen concentration. A redfish temperature probe was disposed in the raceway pond near the outlet to detect the temperature of the microorganism culture. Illumination to the microorganism culture was provided by natural light (i.e., solar radiation). A low profile greenhouse cover with a 30% aluminet shade cloth covered the raceway pond and blocked at least some sunlight while also exposing the culture volume in the raceway pond to at least some sunlight. A fan mounted in the greenhouse cover forced air circulation across the surface of the aqueous microorganism culture and the head space above the surface of the aqueous microorganism culture. Circulation of the culture between the raceway pond portion and the protein skimmer portion resulted in a duty cycle of 5% (i.e., amount of time the microorganism culture was exposed to light over the total circulation time). The pH level was maintained at between about 7.5 and about 8.5 during the test run, with the controls set point at 7.5.

The bioreactor was cleaned and bleach sterilized per the Heliae standard operating procedures prior to the experiment. The bioreactor was inoculated at an initial concentration of 0.08 g/L. Culture media was made using UV treated water and laboratory BG-11 stocks. The media was made to 1 times BG-11 with nitrates and phosphates reduced for outside reactors. The media was nitrogen-sparged to decrease dissolved oxygen (DO) concentration to 3 mg $O_2$ /L (only on the initial inoculation and not thereafter). During inoculation, carboys containing the seed cultures of *Chlorella* were opened and the seed was poured directly into the circulating culture media. It was noticed that immediately after inoculation the DO concentration had reached ~ 9 mg $O_2$ /L. All the usual samples for nutrients and bacteria were collected as previously described in the other examples.

Samples for concentration and nitrate levels were sent to the laboratory daily. Samples were submitted daily for FACs, petrifilm, and microscopy when associates trained to do the aforementioned tests were available. The samples were collected from the south side of the bioreactor system where the sample port in the cover was placed. A log book was completed three times daily. The log book had the following fields: Date, Time, pH, Temp, DO concentration, PAR, ACID refill, Sample taken, Unit volume, Harvest, Initial, and Notes. To maintain DO concentration, the air flow rate into the protein skimmer was increased as the culture cell concentration increased. The protein skimmer unit produced wet foam as it ran, and the protein skimmer discharge valve was fully opened to allow dry white foam to be constantly skimmed and the protein skimmer unit volume to decrease from the previous 946L to 908L. The protein skimmer wash down was set for 25 seconds approximately every 4 hours, with the collection barrel emptied daily. The targeted nitrate level for the reactor for the majority of the run was 700 ppm with daily feedings of sodium nitrate and the proper ratio of potassium phosphate. The target for harvesting was to wait until the culture reached a density of 5 g/L or greater and then harvest from the culture to reduce the density to 2.25 g/L.

Harvesting of the *Chlorella* biomass was performed by pumping out a volume of the culture from the raceway pond equivalent to a harvest of 55% of the total bioreactor system volume. Harvesting periods are noted in the figures below by the vertical dashed lines. UV treated water was then added to replace the culture harvested as well as 1 times BG-11 stocks (Nitrate 700ppm) for the volume of the entire reactor. The concentration in the culture was maintained between 2-5 g/L for the majority of the test run with the concentration reaching 7-8 g/L briefly on two days.

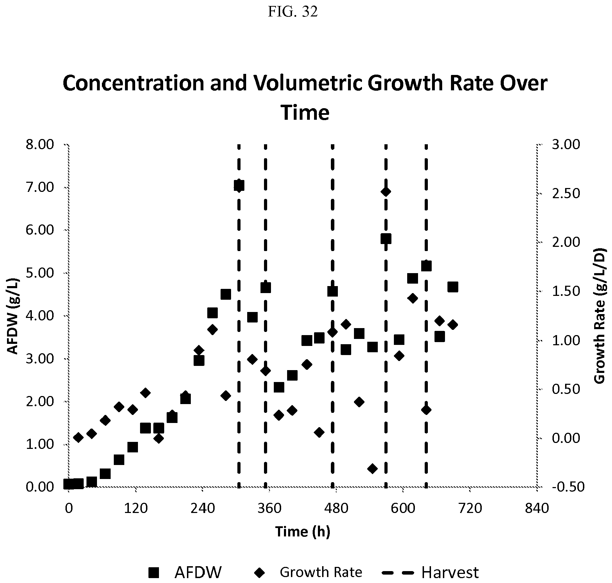

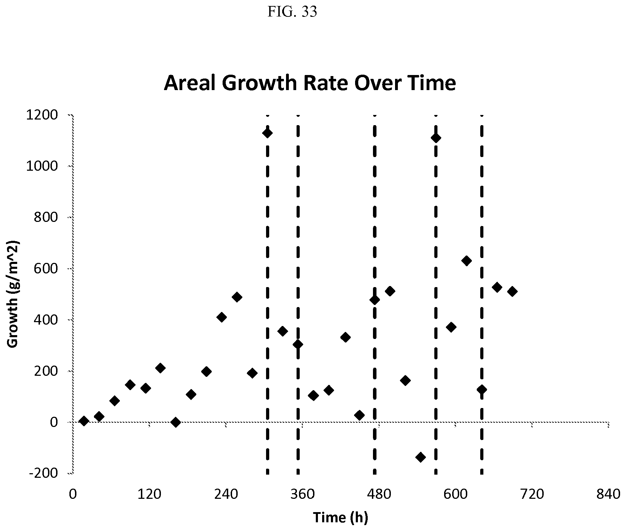

The results for concentration, volumetric growth rate, and areal growth rate over time are shown in FIGS. 32-33 for the test run. The dashed harvest lines in the figures correspond to a 55% (~500L) harvest. Before the first harvest, the increase in concentration exhibits a classic algal growth curve (lag - exponential - stationary).

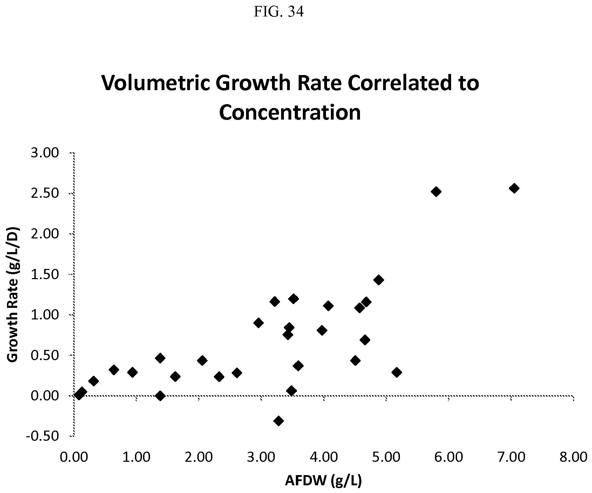

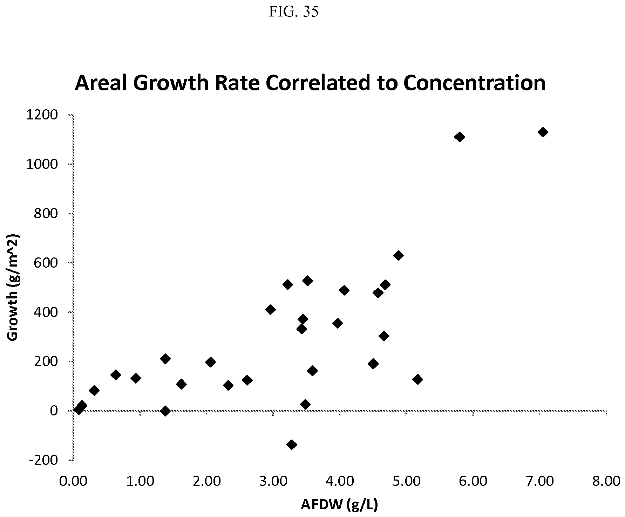

The FIGS. 34-35 show the correlation of concentration (g/L) to volumetric growth rate and areal growth rate for the test run, and may suggest for the bioreactor system that 3 g/L should be the lower threshold when harvesting and operating the reactor. The two points of very high growth occur above a concentration of 5 g/L suggest that further optimization of productivity may be possible with higher operating concentrations.

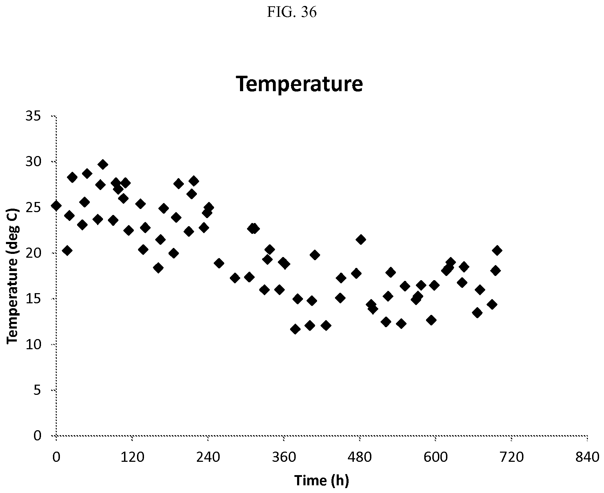

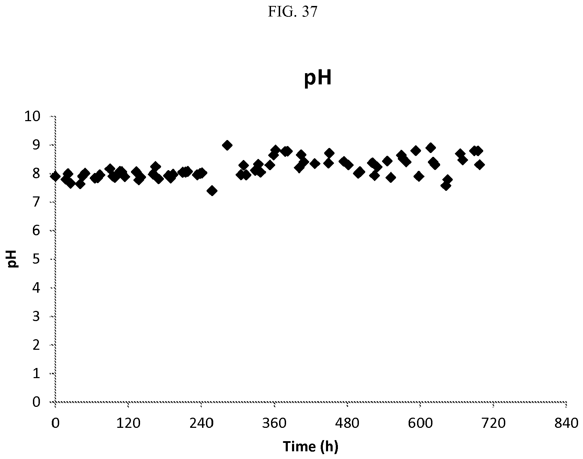

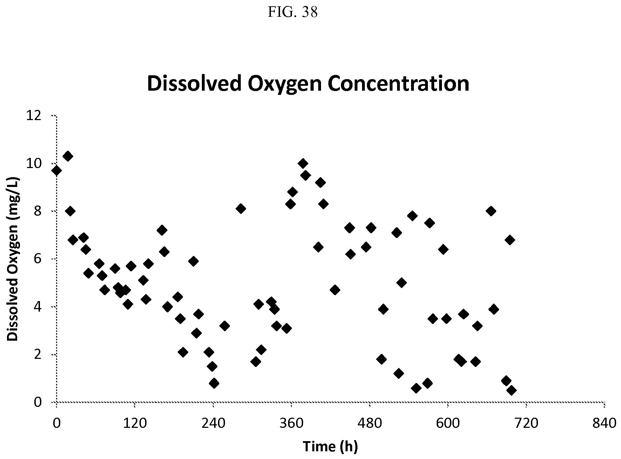

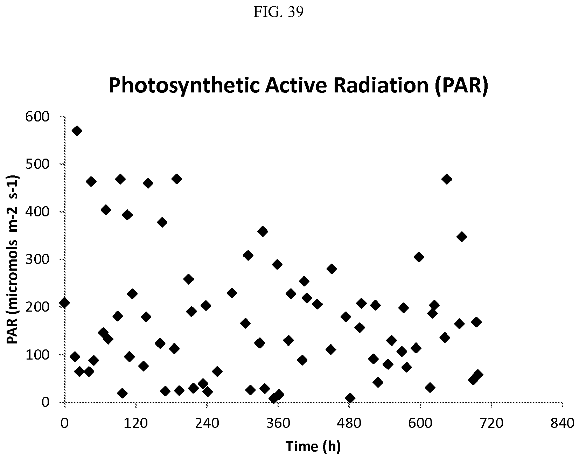

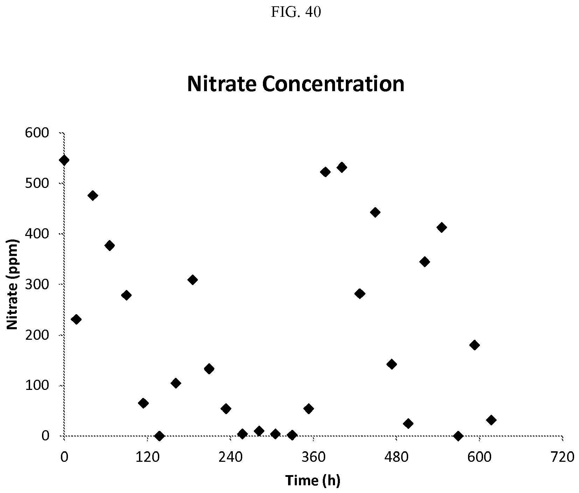

FIGS. 36-40 show the environmental parameters during the culturing run of the mixotrophic bioreactor system, including the temperature, pH level, dissolved oxygen concentration, illumination (i.e., photosynthetic active radiation), and nitrate concentration. In the figure displaying temperature, the results display the thermal stability of the culture volume through the small changes in temperature on a daily basis.

During the run, daily microscope observations were carried out. The observations showed the culture following the same progression as most outdoor cultures, increasing in cell density as well as contamination comprising cell debris and bacteria. However, the *Chlorella* culture in the mixotrophic bioreactor system culture differed from previous reactor runs as the warning signs (e.g., putrid smell, predators, or algae-attacking bacteria) that forecast a culture crash (i.e., dominance by contaminating organisms) were not observed. Acetic acid was used as the organic carbon source, but no antifoam, antibiotics, ozone, or UV were used in the test run. The mixotrophic bioreactor system was not sealed and was operated in non-axenic conditions. The culture continued growth for 34 days, with an average growth rate over the 34 days of 302 g/m$^2$ d.

Organic carbon combinations

While mixotrophic cultures may operate using a supply of a single organic carbon source, some cultures of microorganisms may experience a benefit from the use of a combination of organic carbon sources. The organic carbon supply may comprise at least one organic carbon source. The organic carbon supply may comprise at least two organic carbon sources. A culture of mixotrophic microorganisms may use an organic carbon supply comprising acetic acid and at least one additional organic carbon source.

When culturing microorganisms in mixotrophic conditions using acetic acid as the principal organic carbon source, the excretion of small amounts of succinic acid to the culture media may occur due to metabolic overflow. The excretion of succinic acid may decrease the alkalinity of the medium and displace the residual acetic acid present in the culture medium. Using a pH-auxostat system for dosing acetic acid to a mixotrophic culture of *Chlorella,* it was observed that succinic acid was excreted into the culture medium but acetic acid was still supplied and consumed by the microorganisms, with residual concentrations above 0.5 g acetate/L.

Because succinic acid is an intermediate of the tricarboxylic acid cycle (TCA) that is responsible for metabolizing acetic acid, accumulation of such succinic acid in the culture medium may suggest suboptimal utilization of the organic carbon source in the microorganisms due to some type of metabolic stress or impairment. Energy from acetic acid is produced through the TCA, which is a non-catalytic cycle. Because the TCA is a non-catalytic cycle, the amount of oxaloacetate supplied is dependent on the starting material and not how much acetyl-coA enters the cycle. In a glucose fed microorganism culture, oxaloacetate may be produced from Pyruvate through the Pyruvate Carboxylase; but in an acetic acid supplied microorganism culture, Pyruvate may be supplied through the photosynthesis-glycolysis. The excretion of succinic acid (a TCA intermediate) indicates that photosynthesis may not be able to meet the metabolic demand for oxaloacetate.

It is known in the art that propionic acid may help to recycle the oxaloacetate in an acetate fed dinoflagellate culture (e.g., *Crypthecodinium cohnii). It* is also known in the art that in fungi, propionic acid enters into the TCA after conversion to succinyl-coA or via citramalate. Entering the TCA after conversion to succinyl-coA or via citramalate may increase the supply of oxaloacetate required to activate the TCA, and therefore produce sufficient energy for growth and biosynthesis. Pyruvate may also be converted into oxaloacetate through the enzyme pyruvate carboxylase catalyzing the irreversible carboxylation of pyruvate. Applying this knowledge in the context of an acetic acid fed culture of mixotrophic microorganisms, a method may be created to improve the metabolization of acetic acid in mixotrophic microorganisms.

A small amount of an oxaloacetate promoter may be combined with the acetic acid supplied to a mixotrophic microorganism culture in a culturing vessel to help recycle the oxaloacetate into the TCA, enhance growth, enhance biomass productivity, and reduce the inhibitory effect of one or more inhibitors on the TCA. The oxaloacetate promoter may comprise pyruvate; hexose sugar precursors of pyruvate; propionic acid; and precursors of propionic acid such as odd chain fatty acids, valine, isoleucine, threonine, and methionine. The ratio of acetic acid to oxaloacetate promoter may range from 10:0.01 to 10:2. Acetic acid may comprise acetic acid and its precursors, such as acetate and acetic anhydride.

Example 18

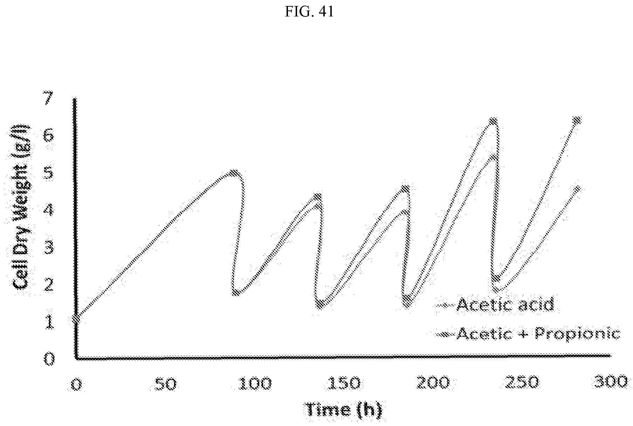

A species of *Chlorella* was cultured in a mixotrophic bioreactor system comprising a pH-auxostat system with 100 g/l of acetic acid and 10 g/l of propionic acid (an acetic acid:propionic acid ratio of 10:1) in the organic carbon source supply container. For the culturing vessel, a bubbled column reactor with 800 ml running volume was utilized. The *Chlorella* was cultured in a semi-continuous mode in which 80 % of the culture was harvested every two days during mixotrophic growth. The culture harvests are shown in FIG. 41 where the trend lines in the graph of the cell dry weight take a sharp decline. Fresh culture medium (BG-11) was added to the culture in order to replace lost culture medium with every harvest.

The cultures were maintained at 25°C and constant aeration of 50 volume air/volume of culture per minute (VVM). Fluorescent light was provided to one side of the culturing vessels at 200 $\mu$mol photon/m$^2$ s in a 24 hour continuous photoperiod. Light path on the culturing vessels was 4 cm. Bacteria levels were maintained below 5 % of total cell counts throughout the experiment.

Four days after inoculation, and after the first semi-continuous harvest, the culture receiving the organic carbon plus promoter treatment of acetic acid:propionic acid (10:1) started to outperform the culture receiving the acetic acid only treatment in terms of cell density and biomass productivity. By the end of the experiment, the culture receiving the organic carbon plus promoter treatment of acetic acid:propionic acid (10:1) showed an increase in productivity of 25 % over the culture receiving acetic acid only. As shown in FIG. 41 and Table 6, the mixture of acetic acid and propionic acid performed as well or better than acetic acid on a daily basis.

TABLE 6

| Organic Carbon Source | Mixotrophic *Chlorella* Daily Productivity (g/L day) | | | | |
|---|---|---|---|---|---|
| | Day1 | Day 2 | Day 3 | Day 4 | Day 5 |
| Acetic Acid | 1.04 | 1.21 | 1.27 | 1.97 | 1.40 |

(continued)

| | Mixotrophic *Chlorella* Daily Productivity (g/L day) | | | | |
|---|---|---|---|---|---|
| Organic Carbon Source | Day1 | Day 2 | Day 3 | Day 4 | Day 5 |
| Acetic Acid + Propionic Acid | 1.05 | 1.34 | 1.54 | 2.33 | 2.21 |

## Claims

1. A method of culturing *Chlorella* in non-axenic mixotrophic culture conditions, comprising:

   (a) inoculating an aqueous culture medium with a culture comprising *Chlorella* in an open culturing vessel;
   (b) supplying light comprising photosynthetically active radiation (PAR) in a photoperiod in the range of 10-16 hours of light for each 24 hour day;
   (c) activating a pH auxostat system with an initial concentration of sodium acetate in the range of 0.05-10 g/L in the aqueous culture medium to start supplying acetic acid, at a concentration in the range of 15-50% (v/v) to the culture comprising *Chlorella,* wherein the pH auxostat system operates with in the range of 6-9, and the temperature ranges from 10 °C to 30 °C; and
   (d) maintaining a dissolved oxygen (DO) concentration in the range of 1-6 mg $O_2$/Liter.

2. The method of claim 1, the method further comprising controlling temperature of the culture with heating and cooling to maintain the temperature within the range of 10-30°C.

3. The method of claim 1, wherein the supplied acetic acid is mixed with a second organic carbon component comprising propionic acid.

4. The method of claim 3, wherein the supplied acetic acid and propionic acid are mixed in an acetic acid:propionic acid ratio in the range of 10:0.01 to 10:2.

5. The method of claim 1, wherein the supplied acetic acid is mixed with at least one additional nutrient comprising at least one selected from the group consisting of nitrates and phosphates.

6. The method of claim 5, wherein the at least one additional nutrient comprises $NO_3$ wherein the acetic acid and $NO_3$ are mixed in an acetic acid: $NO_3$ ratio of 10:0.5 to 10:2.

7. The method of claim 1, the method further comprising harvesting *Chlorella* from the culture on a periodic basis once the density of *Chlorella* in the culture reaches a threshold density of 5 g/L.

8. The method of claim 1, wherein the initial concentration of sodium acetate is in the range of 0.1-6 g/L.

9. The method of claim 1, wherein the initial concentration of sodium acetate is supplied for the first 1-5 days after the culture is inoculated.

## Patentansprüche

1. Verfahren zum Kultivieren von *Chlorella* unter nichtaxenischen mixotrophen Kulturbedingungen, das Folgendes umfasst:

   (a) Inokulieren eines wässrigen Kulturmediums mit einer Kultur umfassend *Chlorella* in einem offenen Kulturgefäß;
   (b) Bereitstellen von Licht umfassend photosynthetisch aktive Strahlung (Photosynthetically Active Radiation, PAR) über eine Photoperiode im Bereich von 10-16 Stunden Licht pro 24-Stunden-Tag;
   (c) Aktivieren eines pH-Auxostat-Systems mit einer anfänglichen Natriumacetatkonzentration im Bereich von 0,05-10 g/l in dem wässrigen Kulturmedium, um mit der Zufuhr von Essigsäure in einer Konzentration im Bereich von 15-50% (v/v) zu der Chlorella-umfassenden Kultur zu beginnen, wobei das pH-Auxostat-System im Bereich von 6-9 tätig ist und die Temperatur im Bereich von 10 °C bis 30 °C liegt; und

(d) Aufrechterhalten einer Gelöst-sauerstoff(Dissolved Oxygen, DO)-Konzentration im Bereich von 1-6 mg $O_2$/Liter.

2. Verfahren nach Anspruch 1, wobei das Verfahren weiterhin Folgendes umfasst: Kontrollieren der Temperatur der Kultur durch Erhitzen und Abkühlen, um die Temperatur im Bereich von 10-30°C aufrechtzuerhalten.

3. Verfahren nach Anspruch 1, wobei die zugeführte Essigsäure mit einer zweiten organischen Kohlenstoffkomponente umfassend Propionsäure vermischt wird.

4. Verfahren nach Anspruch 3, wobei die zugeführte Essigsäure und Propionsäure in einem Essigsäure-Propionsäure-Verhältnis im Bereich von 10:0,01 bis 10:2 vermischt werden.

5. Verfahren nach Anspruch 1, wobei die zugeführte Essigsäure mit mindestens einem zusätzlichen Nährstoff umfassend mindestens einen aus der Gruppe bestehend aus Nitraten und Phosphaten vermischt wird.

6. Verfahren nach Anspruch 5, wobei der mindestens eine zusätzliche Nährstoff $NO_3$ umfasst, wobei die Essigsäure und das $NO_3$ in einem Essigsäure-$NO_3$-Verhältnis von 10:0,5 bis 10:2 vermischt werden.

7. Verfahren nach Anspruch 1, wobei das Verfahren weiterhin Folgendes umfasst: Ernten von *Chlorella* aus der Kultur von Zeit zu Zeit, sobald die *Chlorella*-Dichte in der Kultur einen Dichtegrenzwert von 5 g/l erreicht.

8. Verfahren nach Anspruch 1, wobei die anfängliche Natriumacetatkonzentration im Bereich von 0,1-6 g/l liegt.

9. Verfahren nach Anspruch 1, wobei die anfängliche Natriumacetatkonzentration über die ersten 1-5 Tage nach dem Inokulieren der Kultur bereitgestellt wird.

**Revendications**

1. Méthode de culture de *Chlorella* dans des conditions de culture mixotrophiques non axéniques, comprenant :

(a) l'inoculation d'un milieu de culture aqueux par une culture comprenant *Chlorella* dans un récipient de culture ouvert ;
(b) la fourniture de lumière comprenant des rayonnements photosynthétiquement actifs (RPA) dans une photopériode de l'ordre de 10 à 16 heures de lumière pour chaque journée de 24 heures ;
(c) l'activation d'un système d'auxostat à pH avec une concentration initiale d'acétate de sodium comprise dans l'intervalle 0,05 à 10 g/L dans le milieu de culture aqueux pour commencer la fourniture d'acide acétique, à une concentration de l'ordre de 15 à 50 % en volume, à la culture comprenant *Chlorella,* où le système d'auxostat à pH fonctionne dans la plage allant de 6 à 9, et la température est comprise entre 10 °C et 30 °C ; et
(d) la maintenance d'une concentration en oxygène dissous (OD) de l'ordre de 1 à 6 mg $O_2$/litre.

2. Procédé selon la revendication 1, le procédé comprenant en outre la régulation de la température de la culture en chauffant et en refroidissant pour maintenir la température dans la plage allant de 10 à 30 °C.

3. Procédé selon la revendication 1, où l'acide acétique fourni est mélangé à un deuxième composant carboné organique comprenant de l'acide propionique.

4. Procédé selon la revendication 3, où l'acide acétique et l'acide propionique fournis sont mélangés à un rapport acide acétique/acide propionique de l'ordre de 10:0,01 à 10:2.

5. Procédé selon la revendication 1, où l'acide acétique fourni est mélangé à au moins un nutriment supplémentaire comprenant au moins l'un des membres du groupe constitué par les nitrates et les phosphates.

6. Procédé selon la revendication 5, où l'au moins un nutriment supplémentaire comprend $NO_3$, où l'acide acétique et $NO_3$ sont mélangés dans un rapport acide acétique/ $NO_3$ de 10:0,5 à 10:2.

7. Procédé selon la revendication 1, le procédé comprenant en outre la récolte de *Chlorella* dans la culture sur une base périodique une fois que la densité de *Chlorella* dans la culture atteint un seuil de densité de 5 g/L.

**8.** Procédé selon la revendication 1, où la concentration initiale d'acétate de sodium est comprise dans l'intervalle allant de 0,1 à 6 g/L.

**9.** Procédé selon la revendication 1, où la concentration initiale en acétate de sodium est fournie pendant les premiers 1 à 5 jours après inoculation de la culture.

FIG. 1

FIG. 2

FIG. 3

FIG. 4

FIG. 5

FIG. 6

FIG. 7

FIG. 8

FIG. 9

FIG. 10

FIG. 11

FIG. 12

FIG. 13

FIG. 14

FIG. 15

FIG. 16

FIG. 17

FIG. 18

FIG. 19

FIG. 20

FIG. 21

FIG. 22

FIG. 23

Mixotrophic *Chlorella* Concentration

FIG. 24

**Mixotrophic *Chlorella* Yield**

FIG. 25

**Mixotrophic *Chlorella* Volumetric Growth Rate**

FIG. 26

# Mixotrophic Acetic Acid Consumption

FIG. 27

**Mixotrophic Nitrate Consumption**

FIG. 28

Percentage of Bacteria/*Chlorella*

FIG. 29

FIG. 30

FIG. 31

FIG. 32

Concentration and Volumetric Growth Rate Over Time

FIG. 33

**Areal Growth Rate Over Time**

FIG. 34

**Volumetric Growth Rate Correlated to Concentration**

FIG. 35

Areal Growth Rate Correlated to Concentration

FIG. 36

Temperature

FIG. 37

pH

FIG. 38

**Dissolved Oxygen Concentration**

FIG. 39

Photosynthetic Active Radiation (PAR)

FIG. 40

Nitrate Concentration

FIG. 41

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 61724710 A
- US 61798969 A
- US 61799151 A
- US 61877894 A
- WO 2012109375 A2
- US 3444647 A

**Non-patent literature cited in the description**

- **YUE et al.** *Chemical Engineering Science,* 2007, vol. 62, 2096-2108